# EUROPEAN PATENT SPECIFICATION

(11) **EP 3 849 537 B1**
(45) Date of publication and mention of the grant of the patent: **23.10.2024**
(21) Application number: 19859991.2
(22) Date of filing: 09.09.2019
(51) Int. Cl.: A61K 31/519, A61K 31/18, A61K 31/44, A61K 31/4523, A61K 31/506, A61P 35/00

(54) **COMBINATION THERAPIES**
KOMBINATIONSTHERAPIEN
POLYTHÉRAPIES

(30) Priority: 10.09.2018 US 201862729205 P
(43) Date of publication of application: 21.07.2021
(73) Proprietor: Mirati Therapeutics, Inc., San Diego, CA 92121 (US)
(72) Inventor: ENGSTROM, Lars Daniel, Carlsbad, CA 92009 (US); ARANDA, Ruth Wei, San Diego, CA 92108 (US); OLSON, Peter, San Diego, CA 92122 (US); CHRISTENSEN, James Gail, San Diego, CA 92130 (US); HALLIN, Jill, San Diego, CA 92102 (US)
(74) Representative: Elkington and Fife LLP
(86) International application number: PCT/US2019/050233
(87) International publication number: WO 2020/055758

(56) References cited:
- WO-A1-2017/201161
- WO-A1-2018/140600
- WO-A1-2018/140600
- US-A1- 2013 035 336
- US-A1- 2016 108 019
- US-A1- 2016 166 571
- US-A1- 2019 144 444
- BROWER VICKI: "Cell Cycle Inhibitors Make Progress", JOURNAL OF THE NATIONAL CANCER INSTITUTE, vol. 106, no. 7, 1 July 2014 (2014-07-01), GB, pages 1 - 8, XP055923265, ISSN: 0027-8874, DOI: 10.1093/jnci/dju221
- JAY B. FELL ET AL: "Discovery of Tetrahydropyridopyrimidines as Irreversible Covalent Inhibitors of KRAS-G12C with In Vivo Activity", ACS MEDICINAL CHEMISTRY LETTERS, vol. 9, no. 12, 7 November 2018 (2018-11-07), US, pages 1230 - 1234, XP055680197, ISSN: 1948-5875, DOI: 10.1021/acsmedchemlett.8b00382
- O'LEARY ET AL.: "Treating cancer with selective CDK4/6 inhibitors", CLINICAL ONCOLOGY, 1 July 2016 (2016-07-01), pages 417 - 430, XP055581045

## Description

### FIELD OF THE INVENTION

The present invention relates to combination therapies useful for treating cancer. In particular, the present invention relates to therapeutically effective combinations of a cyclin dependent kinase 4 and/or 6 ("CDK 4/6") inhibitor and a KRas G12C inhibitor, pharmaceutical compositions comprising the inhibitors, kits comprising the compositions, methods of use therefor and medical uses therefor.

### BACKGROUND OF THE INVENTION

Kirsten Rat Sarcoma 2 Viral Oncogene Homolog ("KRas") is a small GTPase and a member of the Ras family of oncogenes. KRas serves as a molecular switch cycling between inactive (GDP-bound) and active (GTP-bound) states to transduce upstream cellular signals received from multiple tyrosine kinases to downstream effectors regulating a wide variety of processes, including cellular proliferation (e.g., see Alamgeer et al., (2013) Current Opin Pharmcol. 13:394-401).

The role of activated KRas in malignancy was observed over thirty years ago (e.g., see Santos et al., (1984) Science 223:661-664). Aberrant expression of KRas accounts for up to 20% of all cancers and oncogenic KRas mutations that stabilize GTP binding and lead to constitutive activation of KRas and downstream signaling have been reported in 25 -30% of lung adenocarcinomas. (e.g., see Samatar and Poulikakos (2014) Nat Rev Drug Disc 13(12): 928-942 doi: 10.1038/nrd428). Single nucleotide substitutions that result in missense mutations at codons 12 and 13 of the KRas primary amino acid sequence comprise approximately 40% of these KRas driver mutations in lung adenocarcinoma, with a G12C transversion being the most common activating mutation (e.g., see Dogan et al., (2012) Clin Cancer Res. 18(22):6169-6177, published online 2012 Sep 26. doi: 10.1158/1078-0432.CCR-11-3265).

The well-known role of KRas in malignancy and the discovery of these frequent mutations in KRas in various tumor types made KRas a highly attractable target of the pharmaceutical industry for cancer therapy. Notwithstanding thirty years of large scale discovery efforts to develop inhibitors of KRas for treating cancer, no KRas inhibitor has demonstrated sufficient safety and/or efficacy to obtain regulatory approval (e.g., see McCormick (2015) Clin Cancer Res. 21 (8): 1797-1801).

Compounds that inhibit KRas activity are still highly desirable and under investigation, including those that disrupt effectors such as guanine nucleotide exchange factors (e.g., see Sun et al., (2012) Agnew Chem Int Ed Engl. 51(25):6140-6143 doi: 10.1002/ame201201358) as well as those that target KRas G12C (e.g., see Ostrem et al., (2013) Nature 503:548-551). Clearly there remains a continued interest and effort to develop inhibitors of KRas, particularly inhibitors of activating KRas mutants, including KRas G12C.

While the KRas G12C inhibitors disclosed herein are potent inhibitors of KRas G12C enzymatic activity and exhibit single agent activity inhibiting the in vitro proliferation of cell lines harboring a KRas G12C mutation, the relative potency and/or observed maximal effect of any given KRas G12C inhibitor can vary between KRAS mutant cell lines. The reason or reasons for the range of potencies and observed maximal effect is not fully understood but certain cell lines appear to possess differing intrinsic resistance. Thus, there is a need to develop alternative approaches to maximize the potency, efficacy, therapeutic index and/or clinical benefit of KRas G12C inhibitors in vitro and in vivo.
WO 2017/201161 relates to compounds that inhibit KRas G12C, in particular compounds that irreversibly inhibit the activity of KRas G12C, pharmaceutical compositions comprising the compounds and methods of use therefor.
WO 2018/140600 relates to compounds having activity as inhibitors of G12C mutant KRAS protein, methods associated with preparation and use of such compounds, pharmaceutical compositions comprising such compounds and methods to modulate the activity of G12C mutant KRAS protein for treatment of disorders, such as cancer.
Vicki Brower, Cell Cycle Inhibitors Make Progress, JNCI: Journal of the National Cancer Institute, Volume 106, Issue 7, July 2014 relates to CDK4/6 inhibitors.

The combination therapy of the present invention, in one aspect, synergistically increases the potency of KRas G12C inhibitors resulting in improved efficacy of KRas G12C inhibitors disclosed herein. The combination therapy of the present invention, in another aspect, provides improved clinical benefit to patients compared to treatment with KRas G12C inhibitors disclosed herein as a single agent.

### SUMMARY OF THE INVENTION

In one aspect of the invention, provided herein is a combination of a CDK 4/6 inhibitor and a KRAS G12C inhibitor for use in a method of treating cancer in a subject in need thereof, comprising administering to the subject a therapeutically effective amount of the combination of a CDK 4/6 inhibitor and a KRAS G12C inhibitor,
wherein the CDK 4/6 inhibitor is abemaciclib or palbociclib, and
wherein the KRas G12C inhibitor is selected from the group consisting of: 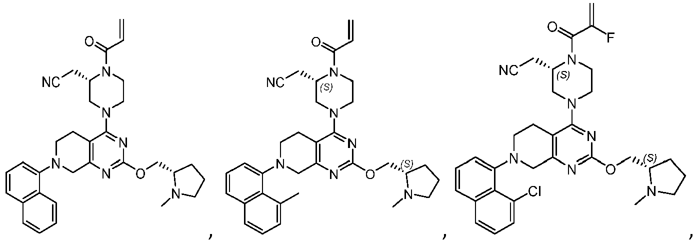 and 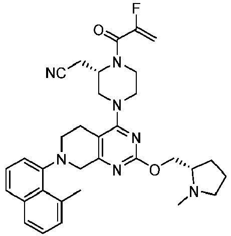 or pharmaceutically acceptable salts thereof.

In another aspect of the invention, pharmaceutical compositions are provided comprising a therapeutically effective amount of a combination of a CDK 4/6 inhibitor of the invention and a KRas G12C inhibitor compound of the invention, or a pharmaceutically acceptable salt thereof and a pharmaceutically acceptable excipient.

In one aspect of the invention, provided herein is a CDK 4/6 inhibitor of the invention or a pharmaceutical composition thereof and a KRAS G12C inhibitor of the invention, or a pharmaceutically acceptable salt or a pharmaceutical composition thereof for use in a method of treating cancer in a subject in need thereof, comprising administering to the subject a therapeutically effective amount of the combination of a CDK 4/6 inhibitor of the invention or a pharmaceutical composition thereof and a KRAS G12C inhibitor of the invention, or a pharmaceutically acceptable salt or a pharmaceutical composition thereof. In one embodiment, the cancer is a KRas G12C-associated cancer. In one embodiment, the KRas G12C-associated cancer is lung cancer.

In some aspects of the invention, KRas G12C inhibitor compounds and CDK 4/6 inhibitors are the only active agents in the provided combinations, methods and medical uses.

Examples of CDK 4/6 inhibitors include palbociclib, abemaciclib, ribociclib, trilaciclib and PF-06873600.

Also provided herein is an in vitro method for increasing the sensitivity of a cancer cell to a KRas G12C inhibitor, comprising contacting the cancer cell with a therapeutically effective amount of a combination of a KRas G12C inhibitor compound of the invention, or a pharmaceutically acceptable salt or a pharmaceutical composition thereof and a CDK 4/6 inhibitor of the invention or a pharmaceutical composition thereof, wherein the CDK 4/6 inhibitor synergistically increases the sensitivity of the cancer cell to the KRas G12C inhibitor. Also provided herein is a combination of a KRas G12C inhibitor compound of the invention, or a pharmaceutically acceptable salt or a pharmaceutical composition thereof and a CDK 4/6 inhibitor of the invention or a pharmaceutical composition thereof for use in a method for increasing the sensitivity of a cancer cell to a KRas G12C inhibitor, comprising contacting the cancer cell with a therapeutically effective amount of the combination of a KRas G12C inhibitor compound of the invention, or a pharmaceutically acceptable salt or a pharmaceutical composition thereof and a CDK 4/6 inhibitor of the invention or a pharmaceutical composition thereof, wherein the CDK 4/6 inhibitor synergistically increases the sensitivity of the cancer cell to the KRas G12C inhibitor.

Also provided herein is a combination of a CDK 4/6 inhibitor of the invention or a pharmaceutical composition thereof and a KRas G12C inhibitor compound of the invention, or a pharmaceutically acceptable salt or a pharmaceutical composition thereof for use in a method for treating cancer in a subject in need thereof, the method comprising (a) determining that cancer is associated with a KRas G12C mutation (e.g., a KRas G12C-associated cancer) (e.g., as determined using a regulatory agency-approved, e.g., FDA-approved, assay or kit); and (b) administering to the patient a therapeutically effective amount of the combination of a CDK 4/6 inhibitor of the invention or a pharmaceutical composition thereof and a KRas G12C inhibitor compound of the invention, or a pharmaceutically acceptable salt or a pharmaceutical composition thereof, wherein the CDK 4/6 inhibitor synergistically increases the sensitivity of the KRas G12C-associated cancer to the KRas G12C inhibitor.

Also provided herein are kits comprising a CDK 4/6 inhibitor of the invention or a pharmaceutical composition thereof and a KRas G12C inhibitor compound of the invention, or a pharmaceutically acceptable salt or a pharmaceutical composition thereof. Also provided is a kit comprising a CDK 4/6 inhibitor of the invention or a pharmaceutical composition thereof and a KRas G12C inhibitor compound of the invention, or a pharmaceutically acceptable salt or a pharmaceutical composition thereof, for use in treating a KRas G12C cancer.

In a related aspect, the invention provides a kit containing a dose of a CDK 4/6 inhibitor of the invention or a pharmaceutical composition thereof and a KRas G12C inhibitor compound of the invention, or a pharmaceutically acceptable salt or a pharmaceutical composition thereof in an amount effective to inhibit proliferation of cancer cells in a subject. The kit in some cases includes an insert with instructions for administration of a CDK 4/6 inhibitor of the invention or a pharmaceutical composition thereof and a KRas G12C inhibitor compound of the invention, or a pharmaceutically acceptable salt or a pharmaceutical composition thereof. The insert may provide a user with one set of instructions for using a CDK 4/6 inhibitor of the invention or a pharmaceutical composition thereof in combination with a KRas G12C inhibitor compound of the invention, or a pharmaceutically acceptable salt or a pharmaceutical composition thereof.

In some embodiments of any of the aspects described herein, before treatment with the compositions of the invention, the patient was treated with one or more of a chemotherapy, a targeted anticancer agent, radiation therapy, and surgery, and optionally, the prior treatment was unsuccessful; and/or the patient has been administered surgery and optionally, the surgery was unsuccessful; and/or the patient has been treated with a platinum-based chemotherapeutic agent, and optionally, the patient has been previously determined to be non-responsive to treatment with the platinum-based chemotherapeutic agent; and/or the patient has been treated with a kinase inhibitor, and optionally, the prior treatment with the kinase inhibitor was unsuccessful; and/or the patient was treated with one or more other therapeutic agent(s). The scope of the invention is defined by the claims. The references to methods of treatment in the summary and detailed description of the invention in this description are to be interpreted as references to the compounds, pharmaceutical compositions and medicaments of the present invention for use in a method for treatment of the human (or animal) body by therapy. Any embodiment not falling under the scope of the claims is provided for information purposes only.

### DETAILED DESCRIPTION OF THE INVENTION

The present invention relates to combination therapies for treating KRas G12C cancers. In particular, the present invention relates to a combination of a CDK 4/6 inhibitor and a KRAS G12C inhibitor for use in a method of treating cancer in a subject in need thereof, comprising administering to the subject a therapeutically effective amount of the combination of a CDK 4/6 inhibitor or a pharmaceutical composition thereof and a KRAS G12C inhibitor, or a pharmaceutically acceptable salt or a pharmaceutical composition thereof, wherein the CDK 4/6 inhibitor is abemaciclib or palbociclib, and wherein the KRas G12C inhibitor is selected from the group consisting of: 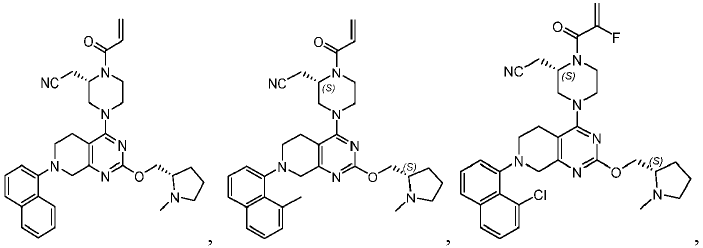 and 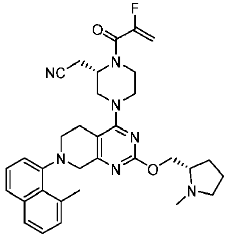 or pharmaceutically acceptable salts thereof, pharmaceutical compositions comprising therapeutically effective amounts of the inhibitors, kits comprising the compositions, methods of use therefor and medical uses therefor.

Combinations of a CDK 4/6 inhibitor of the invention with a KRas G12C inhibitor compound of the invention or pharmaceutically acceptable salts thereof, synergistically increase the potency of the KRas G12C inhibitor compounds of the invention against cancer cells that express KRas G12C thereby increasing the efficacy and therapeutic index of KRas G12C inhibitor compounds of the invention, or pharmaceutically acceptable salts thereof.

### DEFINITIONS

Unless defined otherwise, all technical and scientific terms used herein have the same meaning as is commonly understood by one of skill in the art to which this invention belongs.

As used herein, "KRas G12C" refers to a mutant form of a mammalian KRas protein that contains an amino acid substitution of a cysteine for a glycine at amino acid position 12. The assignment of amino acid codon and residue positions for human KRas is based on the amino acid sequence identified by UniProtKB/Swiss-Prot P01116: Variant p.Gly12Cys.

As used herein, a "KRas G12C inhibitor" refers to compounds of the present invention. These compounds are capable of negatively modulating or inhibiting all or a portion of the enzymatic activity of KRas G12C. The KRas G12C inhibitors of the present invention interact with and irreversibly bind to KRas G12C by forming a covalent adduct with the sulfhydryl side chain of the cysteine residue at position 12 resulting in the inhibition of the enzymatic activity of KRas G12C.

A "KRas G12C-associated disease or disorder" as used herein refers to diseases or disorders associated with or mediated by or having a KRas G12C mutation. A non-limiting example of a KRas G12C-associated disease or disorder is a KRas G12C-associated cancer.

As used herein, "CDK 4/6" refers to members of the mammalian serine/threonine protein kinases CDK 4 and CDK 6 that play key roles in the transition from G1 to S-phase of the cell cycle.

As used herein, a "CDK 4/6 inhibitor" refers to a compound that is capable of negatively modulating or inhibiting all or a portion of the enzymatic activity of CDK 4 and/or 6.

As used herein, the term "subject," "individual," or "patient," used interchangeably, refers to any animal, including mammals such as mice, rats, other rodents, rabbits, dogs, cats, swine, cattle, sheep, horses, primates, and humans. In some embodiments, the patient is a human. In some embodiments, the subject has experienced and/or exhibited at least one symptom of the disease or disorder to be treated and/or prevented. In some embodiments, the subject has been identified or diagnosed as having a cancer having a KRas G12C mutation (e.g., as determined using a regulatory agency-approved, e.g., FDA-approved, assay or kit). In some embodiments, the subject has a tumor that is positive for a KRas G12C mutation (e.g., as determined using a regulatory agency-approved assay or kit). The subject can be a subject with a tumor(s) that is positive for a KRas G12C mutation (e.g., identified as positive using a regulatory agency-approved, e.g., FDA-approved, assay or kit). The subject can be a subject whose tumors have a KRas G12C mutation (e.g., where the tumor is identified as such using a regulatory agency-approved, e.g., FDA-approved, kit or assay). In some embodiments, the subject is suspected of having a KRas G12C gene-associated cancer. In some embodiments, the subject has a clinical record indicating that the subject has a tumor that has a KRas G12C mutation (and optionally the clinical record indicates that the subject should be treated with any of the compositions provided herein).

The term "pediatric patient" as used herein refers to a patient under the age of 16 years at the time of diagnosis or treatment. The term "pediatric" can be further be divided into various subpopulations including: neonates (from birth through the first month of life); infants (1 month up to two years of age); children (two years of age up to 12 years of age); and adolescents (12 years of age through 21 years of age (up to, but not including, the twenty-second birthday)). Berhman RE, Kliegman R, Arvin AM, Nelson WE. Nelson Textbook of Pediatrics, 15th Ed. Philadelphia: W.B. Saunders Company, 1996; Rudolph AM, et al. Rudolph's Pediatrics, 21st Ed. New York: McGraw-Hill, 2002; and Avery MD, First LR. Pediatric Medicine, 2nd Ed. Baltimore: Williams & Wilkins; 1994.

In some embodiments of any of the methods or uses described herein, an assay is used to determine whether the patient has KRas G12C mutation using a sample (e.g., a biological sample or a biopsy sample such as a paraffin-embedded biopsy sample) from a patient (e.g., a patient suspected of having a KRas G12C-associated cancer, a patient having one or more symptoms of a KRas G12C-associated cancer, and/or a patient that has an increased risk of developing a KRas G12C-associated cancer) can include, for example, next generation sequencing, immunohistochemistry, fluorescence microscopy, break apart FISH analysis, Southern blotting, Western blotting, FACS analysis, Northern blotting, and PCR-based amplification (e.g., RT-PCR, quantitative real-time RT-PCR, allele-specific genotyping or ddPCR). As is well-known in the art, the assays are typically performed, e.g., with at least one labelled nucleic acid probe or at least one labelled antibody or antigen-binding fragment thereof.

The term "regulatory agency" is a country's agency for the approval of the medical use of pharmaceutical agents with the country. For example, a non-limiting example of a regulatory agency is the U.S. Food and Drug Administration (FDA).

The term "amino" refers to -NH₂;

The term "acyl" refers to -C(O)CH₃.

The term "alkyl" as employed herein refers to straight and branched chain aliphatic groups having from 1 to 12 carbon atoms, 1-8 carbon atoms 1-6 carbon atoms, or 1-3 carbon atoms which is optionally substituted with one, two or three substituents. Examples of alkyl groups include, without limitation, methyl, ethyl, propyl, isopropyl, butyl, isobutyl, sec-butyl, tert-butyl, pentyl, and hexyl.

The term "haloalkyl" refers to an alkyl chain in which one or more hydrogen has been replaced by a halogen. Examples of haloalkyls are trifluoromethyl, difluoromethyl and fluoromethyl.

The term "haloalkyloxy" refers to -O-haloalkyl.

An "alkylene," group is an alkyl group, as defined hereinabove, that is positioned between and serves to connect two other chemical groups. Exemplary alkylene groups include, without limitation, methylene, ethylene, propylene, and butylene.

The term "alkoxy" refers to -OC1 - C6 alkyl.

The term "cycloalkyl" as employed herein includes saturated and partially unsaturated cyclic hydrocarbon groups having 3 to 12 carbons, for example 3 to 8 carbons, and as a further example 3 to 6 carbons, wherein the cycloalkyl group additionally is optionally substituted. Examples of cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroalkyl" refers to an alkyl group, as defined hereinabove, wherein one or more carbon atoms in the chain are replaced by a heteroatom selected from the group consisting of O, S, and N.

As used herein, the term "hydroxyalkyl" refers to -alkyl-OH.

The term "dihydroxyalkyl" refers to an alkyl group as defined herein wherein two carbon atoms are each substituted with a hydroxyl group.

The term "alkylaminyl" refers to -NR^{x}-alkyl, wherein R^{x} is hydrogen. In one embodiment, R^{x} is hydrogen.

The term "dialkylaminyl" refers to -N(R^{y})₂, wherein each R^{y} is C1 - C3 alkyl.

The term "alkylaminylalkyl" refers to -alkyl-NR^{x}-alkyl, wherein R^{x} is hydrogen. In one embodiment, R^{x} is hydrogen.

The term "dialkylaminylalkyl" refers to -alkyl-N(R^{y})₂, wherein each R^{y} is C1 - C4 alkyl, wherein the alkyl of the--alkyl-N(R^{y})₂ may be optionally substituted with hydroxy or hydroxyalkyl.

An "aryl" group is a C₆-C₁₄ aromatic moiety comprising one to three aromatic rings, which is optionally substituted. As one embodiment, the aryl group is a C₆-C₁₀ aryl group. Examples of aryl groups include, without limitation, phenyl, naphthyl, anthracenyl, fluorenyl, and dihydrobenzofuranyl.

An "aralkyl" or "arylalkyl" group comprises an aryl group covalently linked to an alkyl group, either of which may independently be optionally substituted or unsubstituted. An example of an aralkyl group is (C₁- C₆)alkyl(C₆-C₁₀)aryl, including, without limitation, benzyl, phenethyl, and naphthylmethyl. An example of a substituted aralkyl is wherein the alkyl group is substituted with hydroxyalkyl.

A "heterocyclyl" or "heterocyclic" group is a ring structure having from about 3 to about 12 atoms, for example 4 to 8 atoms, wherein one or more atoms are selected from the group consisting of N, O, and S, the remainder of the ring atoms being carbon. The heterocyclyl may be a monocyclic, a bicyclic, a spirocyclic or a bridged ring system. The heterocyclic group is optionally substituted with R⁷ on carbon or nitrogen at one or more positions, wherein R⁷ is as defined for Formula I. The heterocyclic group is also independently optionally substituted on nitrogen with alkyl, aryl, aralkyl, alkylcarbonyl, alkylsulfonyl, arylcarbonyl, arylsulfonyl, alkoxycarbonyl, aralkoxycarbonyl, or on sulfur with oxo or lower alkyl. Examples of heterocyclic groups include, without limitation, epoxy, azetidinyl, aziridinyl, tetrahydrofuranyl, tetrahydropyranyl, pyrrolidinyl, pyrrolidinonyl, piperidinyl, piperazinyl, imidazolidinyl, thiazolidinyl, dithianyl, trithianyl, dioxolanyl, oxazolidinyl, oxazolidinonyl, decahydroquinolinyl, piperidonyl, 4-piperidinonyl, thiomorpholinyl, thiomorpholinyl 1,1 dioxide, morpholinyl, oxazepanyl, azabicyclohexanes, azabicycloheptanes and oxa azabiocycloheptanes. Specifically excluded from the scope of this term are compounds having adjacent annular O and/or S atoms.

The term "heterocyclylalkyl" refers to a heterocyclyl group as defined herein linked to the remaining portion of the molecule via an alkyl linker, wherein the alkyl linker of the heterocyclylalkyl may be optionally substituted with hydroxy or hydroxyalkyl.

As used herein, the term "heteroaryl" refers to groups having 5 to 14 ring atoms, preferably 5, 6, 9, or 10 ring atoms; having 6, 10, or 14 π electrons shared in a cyclic array; and having, in addition to carbon atoms, from one to three heteroatoms per ring selected from the group consisting of N, O, and S. Examples of heteroaryl groups include acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, furanyl, furazanyl, imidazolinyl, imidazolyl, 1H-indazolyl, indolenyl, indolinyl, indolizinyl, indolyl, 3H-indolyl, isobenzofuranyl, isochromanyl, isoindazolyl, isoindolinyl, isoindolyl, isoquinolinyl, isothiazolyl, isoxazolyl, methylenedioxyphenyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolinyl, 2H-pyrrolyl, pyrrolyl, quinazolinyl, quinolinyl, 4H-quinolizinyl, quinoxalinyl, quinuclidinyl, tetrahydroisoquinolinyl, tetrahydroquinolinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

A "heteroarylalkyl" group comprises a heteroaryl group covalently linked to an alkyl group, wherein the radical is on the alkyl group, either of which is independently optionally substituted or unsubstituted. Examples of heteroarylalkyl groups include a heteroaryl group having 5, 6, 9, or 10 ring atoms bonded to a C1-C6 alkyl group. Examples of heteroaralkyl groups include pyridylmethyl, pyridylethyl, pyrrolylmethyl, pyrrolylethyl, imidazolylmethyl, imidazolylethyl, thiazolylmethyl, thiazolylethyl, benzimidazolylmethyl, benzimidazolylethyl quinazolinylmethyl, quinolinylmethyl, quinolinylethyl, benzofuranylmethyl, indolinylethyl isoquinolinylmethyl, isoinodylmethyl, cinnolinylmethyl, and benzothiophenylethyl. Specifically excluded from the scope of this term are compounds having adjacent annular O and/or S atoms.

As used herein, "an effective amount" of a compound is an amount that is sufficient to negatively modulate or inhibit the activity of the desired target, i.e., a CDK 4/6 or KRas G12C. Such amount may be administered as a single dosage or may be administered according to a regimen, whereby it is effective.

As used herein, a "therapeutically effective amount" of a compound is an amount that is sufficient to ameliorate, or in some manner reduce a symptom or stop or reverse progression of a condition, or negatively modulate or inhibit the activity of CDK 4/6s or KRas G12C. Such amount may be administered as a single dosage or may be administered according to a regimen, whereby it is effective.

As used herein, a "therapeutically effective amount of a combination" of two compounds is an amount that together synergistically increases the activity of the combination in comparison to the therapeutically effective amount of each compound in the combination, i.e., more than merely additive. Alternatively, in vivo, the therapeutically effective amount of the combination of a CDK 4/6 inhibitor of the invention or a pharmaceutical composition thereof and a KRas G12C inhibitor compound of the invention, or a pharmaceutically acceptable salt or a pharmaceutical composition thereof, results in an increased duration of overall survival ("OS") in subjects relative to treatment with only the KRas G12C inhibitor. In one embodiment, the therapeutically effective amount of the combination of a CDK 4/6 inhibitor of the invention or a pharmaceutical composition thereof and a KRas G12C inhibitor compound of the invention, or a pharmaceutically acceptable salt or a pharmaceutical composition thereof, results in an increased duration of progression-free survival ("PFS") in subjects relative to treatment with only the KRas G12C inhibitor. In one embodiment, the therapeutically effective amount of the combination of a CDK 4/6 inhibitor of the invention or a pharmaceutical composition thereof and a KRas G12C inhibitor compound of the invention, or a pharmaceutically acceptable salt or a pharmaceutical composition thereof, results in increased tumor regression in subjects relative to treatment with only the KRas G12C inhibitor. In one embodiment, the therapeutically effective amount of the combination of a CDK 4/6 inhibitor of the invention or a pharmaceutical composition thereof and a KRas G12C inhibitor compound of the invention, or a pharmaceutically acceptable salt or a pharmaceutical composition thereof, results in increased tumor growth inhibition in subjects relative to treatment with only the KRas G12C inhibitor. In one embodiment, the therapeutically effective amount of the combination of a CDK 4/6 inhibitor of the invention or a pharmaceutical composition thereof and a KRas G12C inhibitor compound of the invention, or a pharmaceutically acceptable salt or a pharmaceutical composition thereof, results in an improvement in the duration of stable disease in subjects compared to treatment with only the KRas G12C inhibitor. The amount of each compound in the combination may be the same or different than the therapeutically effective amount of each compound when administered alone as a monotherapy as long as the combination is synergistic. Such amounts may be administered as a single dosage or may be administered according to a regimen, whereby it is effective.

As used herein, treatment means any manner in which the symptoms or pathology of a condition, disorder or disease are ameliorated or otherwise beneficially altered. Treatment also encompasses any pharmaceutical use of the compositions herein.

As used herein, amelioration of the symptoms of a particular disorder by administration of a particular pharmaceutical composition refers to any lessening, whether permanent or temporary, lasting or transient that can be attributed to or associated with administration of the composition.

As used herein, the term "about" when used to modify a numerically defined parameter (e.g., the dose of a KRAS inhibitor or a CDK 4/6 inhibitor or a pharmaceutically acceptable salt thereof, or the length of treatment time with a combination therapy described herein) means that the parameter may vary by as much as 10% below or above the stated numerical value for that parameter. For example, a dose of about 5 mg/kg may vary between 4.5 mg/kg and 5.5 mg/kg. "About" when used at the beginning of a listing of parameters is meant to modify each parameter. For example, about 0.5 mg, 0.75 mg or 1.0 mg means about 0.5 mg, about 0.75 mg or about 1.0 mg. Likewise, about 5% or more, 10% or more, 15% or more, 20% or more, and 25% or more means about 5% or more, about 10% or more, about 15% or more, about 20% or more, and about 25% or more.

### INHIBITOR COMPOUNDS

In one aspect of the invention, provided herein is a combination of a CDK 4/6 inhibitor and a KRAS G12C inhibitor for use in a method of treating cancer in a subject in need thereof, comprising administering to the subject a therapeutically effective amount of the combination of a CDK 4/6 inhibitor and a KRAS G12C inhibitor, wherein the CDK 4/6 inhibitor is abemaciclib or palbociclib, and wherein the KRas G12C inhibitor is selected from the group consisting of: 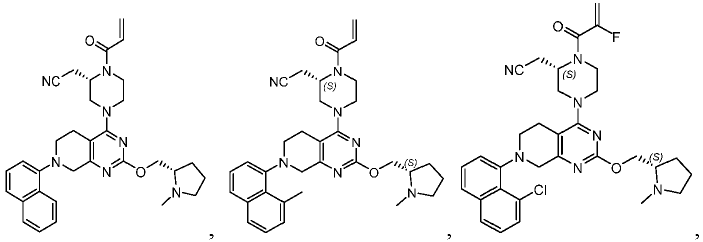 and 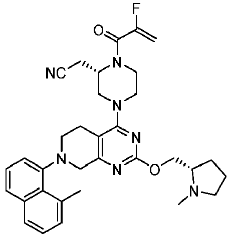 or pharmaceutically acceptable salts thereof.

### 1. CDK 4/6 Inhibitors

The cyclin dependent kinases ("CDK") 4 and 6 are serine/threonine protein kinases that play key roles in the transition from G1 to S-phase of the cell cycle. Both CDK4 and CDK6, in conjunction with their cognate cyclin partners, regulate this transition by phosphorylating the tumor suppressor retinoblastoma ("Rb"), which regulates cell cycle progression. The phosphorylation of Rb disrupts the association between Rb and E2F transcription factors, driving expression of E2F regulated genes whose products are necessary for DNA replication in S-phase (for a review see, for example, de Groot et al., Cancer Treat Rev. 2017 Nov;60:130-138. doi: 10.1016/j.ctrv.2017.09.003. Epub 2017 Sep 20).

Amplification of CDK4 and/or CDK6 has been reported in a number of tumor types, including breast, sarcomas, gliomas and non-small cell lung cancer. Overexpression of CDK6, for example, results in resistance to temozolomide treatment and hormone therapy treatments, e.g., fulvestrant, in breast cancer patients.

In addition, the activity of CDK4 and CDK6 is negatively regulated by the cyclin dependent kinase inhibitor p16 which is encoded by the CDKN2A gene. CDKN2A normally functions to inhibits CDK4/6 kinase activity thereby preventing phosphorylation of Rb. As a result, Rb remains bound in complex with the E2F transcription factors repressing expression of E2F-regulated gene products, blocking the transition from G1 to S-phase and inhibiting cell proliferation. Inactivating mutations in CDKN2A, particularly homozygous deletions, result in enhanced CDK4/6 activity and unchecked cell cycle progression. Such inactivating mutations and deletions have been reported in several cancer types including bladder, melanoma, glioma, pancreatic, colorectal and non-small cell lung cancer. As such, CDK 4/6 inhibition has gained interest as an approach for anti-cancer therapies beyond the current FDA approved indication of hormone receptor-positive, human epidermal growth factor receptor 2-negative advanced or metastatic breast cancer.

Several inhibitors exhibiting activity against CDK 4/6 have been developed and a number have received marketing approval. For example, approved CDK 4/6 inhibitors include: abemaciclib (N-(5-((4-ethylpiperazin-1-yl)methyl)pyridin-2-yl)-5-fluoro-4-(4-fluoro-1-isopropyl-2-methyl-1H-benzo[d]imidazol-6-yl)pyrimidin-2-amine); palbociclib (6-acetyl-8-cyclopentyl-5-methyl-2-((5-(piperazin-1-yl)pyridin-2-yl)amino)pyrido[2,3-d]pyrimidin-7(8H)-one) and ribociclib (7-cyclopentyl-N,N-dimethyl-2-((5-(piperazin-1-yl)pyridin-2-yl)amino)-7H-pyrrolo[2,3-d]pyrimidine-6-carboxamide) whereas the CDK 4/6 inhibitor trilaciclib (2'-((5-(piperazin-1-yl)pyridin-2-yl)amino)-7',8'-dihydro-6'H-spiro[cyclohexane-1,9'-pyrazino[1',2': 1,5]pyrrolo[2,3-d]pyrimidin]-6'-one) is in late stage clinical trials. Another CDK 4/6 inhibitor useful in the methods herein is the CDK 2/4/6 inhibitor PF-06873600 (Pyrido[2,3-d]pyrimidin-7(8H)-one, 6-(difluoromethyl)-8-[(1R,2R)-2-hydroxy-2-methylcyclopentyl]-2-[[1-(methylsulfonyl)-4-piperidinyl]amino]).

Methods for manufacturing CDK 4/6 inhibitors are well known to those skilled in the art and CDK 4/6 inhibitors may be obtained from a wide variety of commercial suppliers, in forms suitable for both research or human use. In addition, CDK 4/6 inhibitors are disclosed in US Patent Application Publication Nos: US20180201619; US20180201618; US 20180148431; US20170218018; US20170157212; US20170057971; US20150246926; US20150246925; US20150031880; US20150011730; US20140296484; US20140227222; US20140142306; US20140142299; US20130289240; US20130237544; US20130237534; US20130237533; US20130237495; US20130184288; US20130045993; US20120295948; US20120165335; US20110294838; US20110257365; US20110251222; US20110224222; US20110009353; US20100280065; US20100143384; US20080249025; and US20080081811.

### 2. KRas G12C Inhibitors

In the present invention, the KRas G12C inhibitor is selected from the group consisting of: 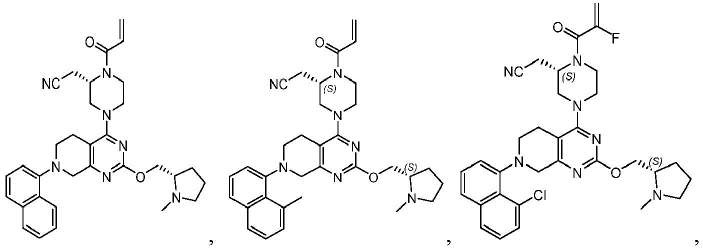 and 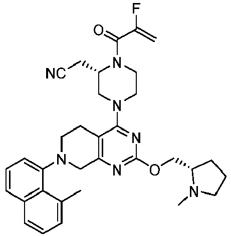 or pharmaceutically acceptable salts thereof.

In the present invention, the KRas G12C inhibitor is selected from: 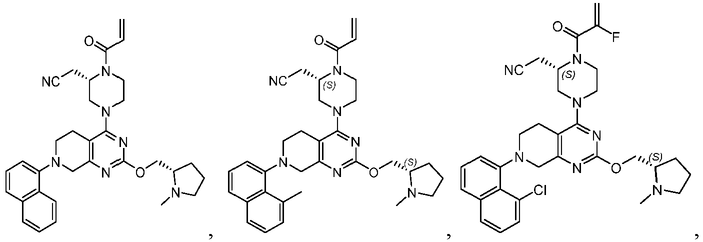 and 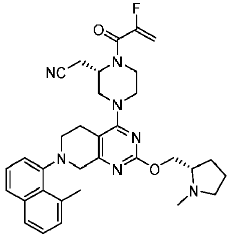 and pharmaceutically acceptable salts thereof.

In one embodiment, the KRas G12C inhibitor is: 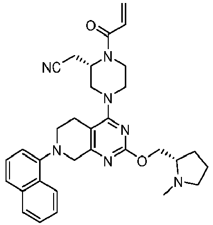 (also referred to as Example 234) and pharmaceutically acceptable salts thereof.

In one embodiment, the KRas G12C inhibitor is: 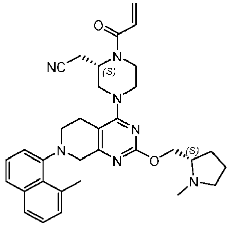 (also referred to as Example 359) and pharmaceutically acceptable salts thereof.

In one embodiment, the KRas G12C inhibitor is: 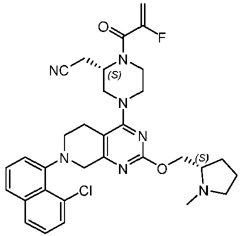 (also referred to as Example 478) and pharmaceutically acceptable salts thereof.

In one embodiment, the KRas G12C inhibitor is: 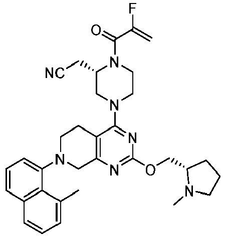 (also referred to as Example 507) and pharmaceutically acceptable salts thereof.

The KRas G12C inhibitors for use in the methods of the present invention may have one or more chiral center and may be synthesized as stereoisomeric mixtures, isomers of identical constitution that differ in the arrangement of their atoms in space. The compounds may be used as mixtures or the individual components/isomers may be separated using commercially available reagents and conventional methods for isolation of stereoisomers and enantiomers well-known to those skilled in the art, e.g., using CHIRALPAK^{®} (Sigma-Aldrich) or CHIRALCEL^{®} (Diacel Corp) chiral chromatographic HPLC columns according to the manufacturer's instructions. Alternatively, compounds of the present invention may be synthesized using optically pure, chiral reagents and intermediates to prepare individual isomers or enantiomers. Unless otherwise indicated, all chiral (enantiomeric and diastereomeric) and racemic forms are within the scope of the invention. Unless otherwise indicated, whenever the specification, including the claims, refers to compounds of the invention, the term "compound" is to be understood to encompass all chiral (enantiomeric and diastereomeric) and racemic forms.

In one embodiment, the KRas G12C inhibitor compounds of the invention for use in the methods include trifluoroacetic acid salts of the above compounds.

Methods for manufacturing the KRas G12C inhibitors disclosed herein are known. For example, commonly owned published international PCT application numbers WO2017201161, and WO2019099524 describe general reaction schemes for preparing KRas G12C inhibitor compounds of the invention and also provide detailed synthetic routes for the preparation of each KRas G12C inhibitor disclosed herein.

The CDK 4/6 inhibitors of the invention and the KRas G12C compounds of the invention, or pharmaceutically acceptable salts thereof may be formulated into pharmaceutical compositions.

### PHARMACEUTICAL COMPOSITIONS

In another aspect, the invention provides pharmaceutical compositions comprising a CDK 4/6 inhibitor and KRas G12C inhibitor according to the invention and a pharmaceutically acceptable carrier, excipient, or diluent that may be used in the methods disclosed herein. The CDK 4/6 inhibitor and KRas G12C inhibitor may be independently formulated by any method well known in the art and may be prepared for administration by any route, including, without limitation, parenteral, oral, sublingual, transdermal, topical, intranasal, intratracheal, or intrarectal. In certain embodiments, CDK 4/6 inhibitor and/or KRas G12C inhibitor are administered intravenously in a hospital setting. In one embodiment, administration may be by the oral route.

The characteristics of the carrier will depend on the route of administration. As used herein, the term "pharmaceutically acceptable" means a non-toxic material that is compatible with a biological system such as a cell, cell culture, tissue, or organism, and that does not interfere with the effectiveness of the biological activity of the active ingredient(s). Thus, compositions may contain, in addition to the inhibitor, diluents, fillers, salts, buffers, stabilizers, solubilizers, and other materials well known in the art. The preparation of pharmaceutically acceptable formulations is described in, e.g., Remington's Pharmaceutical Sciences, 18th Edition, ed. A. Gennaro, Mack Publishing Co., Easton, Pa., 1990.

As used herein, the term pharmaceutically acceptable salt refers to salts that retain the desired biological activity of the above-identified compounds and exhibit minimal or no undesired toxicological effects. Examples of such salts include, but are not limited to acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalacturonic acid. The compounds can also be administered as pharmaceutically acceptable quaternary salts known by those skilled in the art, which specifically include the quaternary ammonium salt of the formula --NR+Z-, wherein R is hydrogen, alkyl, or benzyl, and Z is a counterion, including chloride, bromide, iodide, --O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

The active compound is included in the pharmaceutically acceptable carrier or diluent in an amount sufficient to deliver to a patient a therapeutically effective amount without causing serious toxic effects in the patient treated. In one embodiment, a dose of the active compound for all of the above-mentioned conditions is in the range from about 0.01 to 300 mg/kg, for example 0.1 to 100 mg/kg per day, and as a further example 0.5 to about 25 mg per kilogram body weight of the recipient per day. A typical topical dosage will range from 0.01-3% wt/wt in a suitable carrier. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art.

The pharmaceutical compositions comprising a CDK 4/6 inhibitor and a KRas G12C inhibitor may be for use in the methods described herein.

### CO-ADMINS TRATION

The CDK 4/6 inhibitor, or a pharmaceutically acceptable salt or a pharmaceutically composition thereof and the KRas G12C inhibitor, or a pharmaceutically acceptable salt or a pharmaceutically composition thereof can be formulated into separate or individual dosage forms which can be co-administered one after the other. Another option is that if the route of administration is the same (e.g. oral) two active compounds can be formulated into a single form for co-administration, both methods of co-administration, however, being part of the same therapeutic treatment or regimen.

The pharmaceutical compositions comprising a CDK 4/6 inhibitor and/or a KRas G12C inhibitor, or a pharmaceutically acceptable salt or a pharmaceutically composition thereof for use in the methods may be for simultaneous, separate or sequential use. In one embodiment, the CDK 4/6 inhibitor of the invention or a pharmaceutically composition thereof is administered prior to administration of the KRas G12C inhibitor compound of the invention, or a pharmaceutically acceptable salt or a pharmaceutically composition thereof. In another embodiment, the CDK 4/6 inhibitor of the invention or a pharmaceutically composition thereof is administered after administration of the KRas G12C inhibitor compound of the invention, or a pharmaceutically acceptable salt or a pharmaceutically composition thereof. In another embodiment, the CDK 4/6 inhibitor of the invention or a pharmaceutically composition thereof is administered at about the same time as administration of the KRas G12C inhibitor compound of the invention, or a pharmaceutically acceptable salt or a pharmaceutically composition thereof.

Separate administration of each inhibitor, at different times and by different routes, in some cases would be advantageous. Thus, the components in the combination i.e. the KRas G12C inhibitor compound of the invention, or a pharmaceutically acceptable salt or a pharmaceutically composition thereof and the CDK 4/6 inhibitor of the invention or a pharmaceutically composition thereof, need not be necessarily administered at essentially the same time or in any order. In one embodiment, the CDK 4/6 inhibitor and the KRAS G12C inhibitor are administered on the same day. In one embodiment, the CDK 4/6 inhibitor and the KRAS G12C inhibitor are administered on different days.

Oncology drugs are typically administered at the maximum tolerated dose ("MTD"), which is the highest dose of drug that does not cause unacceptable side effects. In one embodiment, the KRas G12C inhibitor, or a pharmaceutically acceptable salt or a pharmaceutically composition thereof and the CDK 4/6 inhibitor or a pharmaceutically composition thereof are each dosed at their respective MTDs. In one embodiment, the KRas G12C inhibitor, or a pharmaceutically acceptable salt or a pharmaceutically composition thereof is dosed at its MTD and the CDK 4/6 inhibitor or a pharmaceutically composition thereof is dosed in an amount less than its MTD. In one embodiment, the KRas G12C inhibitor, or a pharmaceutically acceptable salt or a pharmaceutically composition thereof is dosed at an amount less than its MTD and the CDK 4/6 inhibitor or a pharmaceutically composition thereof is dosed at its MTD. In one embodiment, the KRas G12C inhibitor, or a pharmaceutically acceptable salt or a pharmaceutically composition thereof and the CDK 4/6 inhibitor or a pharmaceutically composition thereof are each dosed at less than their respective MTDs. The administration can be so timed that the peak pharmacokinetic effect of one compound coincides with the peak pharmacokinetic effect of the other.

In one embodiment, a single dose of KRas G12C inhibitor compound of the invention, or a pharmaceutically acceptable salt or a pharmaceutically composition thereof is administered per day (i.e., in about 24 hour intervals) (i.e., QD). In another embodiment, two doses of the KRas G12C inhibitor compound of the invention, or a pharmaceutically acceptable salt or a pharmaceutically composition thereof are administered per day (i.e., BID). In another embodiment, three doses of the KRas G12C inhibitor compound of the invention, or a pharmaceutically acceptable salt or a pharmaceutically composition thereof are administered per day (i.e., TID).

In one embodiment, the CDK 4/6 inhibitor or a pharmaceutically composition thereof is administered QD. In another embodiment, the CDK 4/6 inhibitor or a pharmaceutically composition thereof are administered BID. In another embodiment, the CDK 4/6 inhibitor or a pharmaceutically composition thereof of the invention are administered TID.

In one embodiment, a single dose of KRas G12C inhibitor compound of the invention, or a pharmaceutically acceptable salt or a pharmaceutically composition thereof and CDK 4/6 inhibitor or a pharmaceutically composition thereof are each administered once daily.

Exemplary CDK 4/6 inhibitors include: abemaciclib (N-(5-((4-ethylpiperazin-1-yl)methyl)pyridin-2-yl)-5-fluoro-4-(4-fluoro-1-isopropyl-2-methyl-1H-benzo[d]imidazol-6-yl)pyrimidin-2-amine); palbociclib (6-acetyl-8-cyclopentyl-5-methyl-2-((5-(piperazin-1-yl)pyridin-2-yl)amino)pyrido[2,3-d]pyrimidin-7(8H)-one) and ribociclib (7-cyclopentyl-N,N-dimethyl-2-((5-(piperazin-1-yl)pyridin-2-yl)amino)-7H-pyrrolo[2,3-d]pyrimidine-6-carboxamide),trilaciclib (2'-((5-(piperazin-1-yl)pyridin-2-yl)amino)-7',8'-dihydro-6'H-spiro[cyclohexane-1,9'-pyrazino[1',2':1,5]pyrrolo[2,3-d]pyrimidin]-6'-one) and PF-06873600 (Pyrido[2,3-d]pyrimidin-7(8H)-one, 6-(difluoromethyl)-8-[(1R,2R)-2-hydroxy-2-methylcyclopentyl]-2-[[1-(methylsulfonyl)-4-piperidinyl]amino]).

### COMBINATION THERAPIES

In one aspect of the invention, provided herein is a combination of a CDK 4/6 inhibitor and a KRAS G12C inhibitor for use in a method of treating cancer in a subject in need thereof, comprising administering to the subject a therapeutically effective amount of the combination of a CDK 4/6 inhibitor and a KRAS G12C inhibitor, wherein the CDK 4/6 inhibitor is abemaciclib or palbociclib, and wherein the KRas G12C inhibitor is selected from the group consisting of: 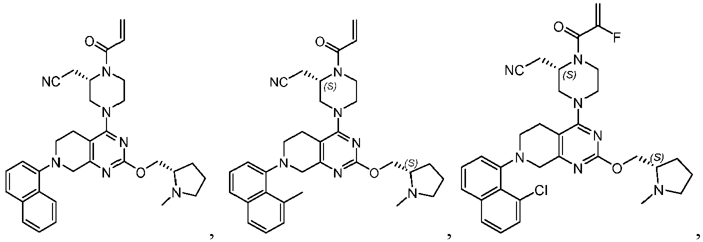 and 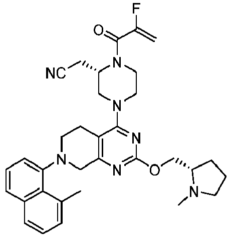 or pharmaceutically acceptable salts thereof. In one embodiment, the cancer is a KRas G12C-associated cancer. In one embodiment, the KRas G12C-associated cancer is lung cancer.

Provided herein is an in vitro method for increasing the sensitivity of a cancer cell to a KRas G12C inhibitor, comprising contacting the cancer cell with an effective amount of a combination of a KRas G12C inhibitor compound of the invention and a CDK 4/6 inhibitor of the invention or pharmaceutical compositions thereof, wherein the CDK 4/6 inhibitor synergistically increases the sensitivity of the cancer cell to the KRas G12C inhibitor. Also provided herein is a combination of a KRas G12C inhibitor compound of the invention and a CDK 4/6 inhibitor of the invention or pharmaceutical compositions thereof for use in a method for increasing the sensitivity of a cancer cell to a KRas G12C inhibitor, comprising contacting the cancer cell with an effective amount of the combination of a KRas G12C inhibitor compound of the invention and a CDK 4/6 inhibitor of the invention or pharmaceutical compositions thereof, wherein the CDK 4/6 inhibitor synergistically increases the sensitivity of the cancer cell to the KRas G12C inhibitor.

In one embodiment, the combination therapy comprises a combination of a compound having the formula: 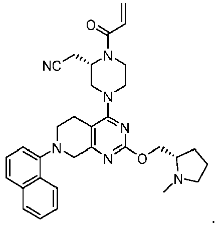 or a pharmaceutically acceptable salt thereof, and a CDK 4/6 inhibitor of the invention. In one embodiment, the CDK 4/6 inhibitor is palbociclib. In one embodiment, the CDK 4/6 inhibitor is abemaciclib.

In one embodiment, the combination therapy comprises a combination of a compound having the formula: 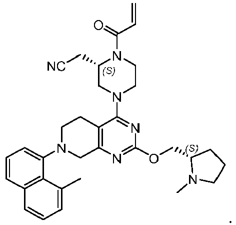 or a pharmaceutically acceptable salt thereof, and a CDK 4/6 inhibitor of the invention. In one embodiment, the CDK 4/6 inhibitor is palbociclib. In one embodiment, the CDK 4/6 inhibitor is abemaciclib.

In one embodiment, the combination therapy comprises a combination of a compound having the formula: 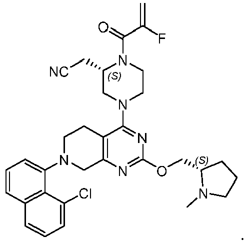 or a pharmaceutically acceptable salt thereof, and a CDK 4/6 inhibitor of the invention. In one embodiment, the CDK 4/6 inhibitor is palbociclib. In one embodiment, the CDK 4/6 inhibitor is abemaciclib.

In one embodiment, the combination therapy comprises a combination of a compound having the formula: 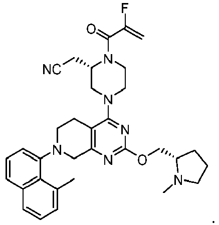 or a pharmaceutically acceptable salt thereof, and a CDK 4/6 inhibitor of the invention. In one embodiment, the CDK 4/6 inhibitor is palbociclib. In one embodiment, the CDK 4/6 inhibitor is abemaciclib.

As used herein, the term "contacting" refers to the bringing together of indicated moieties in an in vitro system or an in vivo system. For example, "contacting" a cancer cell includes the administration of a combination provided herein to an individual or subject, such as a human, having KRas G12C, as well as, for example, introducing a combination provided herein into a sample containing a cellular or purified preparation containing KRas G12C.

By negatively modulating the activity of KRas G12C, the methods described herein are designed to inhibit undesired cellular proliferation resulting from enhanced KRas G12C activity within the cell. The degree of covalent modification of KRas G12C may be monitored in vitro using well known methods, including those described in published international PCT application numbers WO2017201161 and WO2019099524. In addition, the inhibitory activity of combination in cells may be monitored, for example, by measuring the inhibition of KRas G12C activity of the amount of phosphorylated ERK to assess the effectiveness of treatment and dosages may be adjusted accordingly by the attending medical practitioner.

Provided herein is a combination of a CDK 4/6 inhibitor of the invention, or a pharmaceutically acceptable salt or a pharmaceutically composition thereof and a KRas G12C inhibitor compound of the invention, or a pharmaceutically acceptable salt or a pharmaceutical composition thereof for use in the treatment of a KRas G12C-associated cancer in a subject in need thereof, comprising administering to said subject a therapeutically effective amount of the combination of a CDK 4/6 inhibitor of the invention or a pharmaceutically composition thereof and a KRas G12C inhibitor compound of the invention, or a pharmaceutically acceptable salt or a pharmaceutical composition thereof, wherein the CDK 4/6 inhibitor synergistically increases the sensitivity of the Kras G12C-associated cancer to the Kras G12C inhibitor. In one embodiment, the Kras G12C-associated cancer is lung cancer.

In one embodiment, the therapeutically effective amount of the combination of a CDK 4/6 inhibitor of the invention or a pharmaceutically composition thereof and a KRas G12C inhibitor compound of the invention, or a pharmaceutically acceptable salt or a pharmaceutical composition thereof, results in an increased duration of overall survival ("OS") in subjects relative to treatment with only the KRas G12C inhibitor. In one embodiment, the therapeutically effective amount of the combination of a CDK 4/6 inhibitor of the invention or a pharmaceutically composition thereof and a KRas G12C inhibitor compound of the invention, or a pharmaceutically acceptable salt or a pharmaceutical compositions thereof, results in an increased duration of progression-free survival ("PFS") in subjects relative to treatment with only the KRas G12C inhibitor. In one embodiment, the therapeutically effective amount of the combination of a CDK 4/6 inhibitor of the invention or a pharmaceutically composition thereof and a KRas G12C inhibitor compound of the invention, or a pharmaceutically acceptable salt or a pharmaceutical composition thereof, results in increased tumor regression in subjects relative to treatment with only the KRas G12C inhibitor. In one embodiment, the therapeutically effective amount of the combination of a CDK 4/6 inhibitor of the invention or a pharmaceutically composition thereof and a KRas G12C inhibitor compound of the invention, or a pharmaceutically acceptable salt or a pharmaceutical composition thereof, results in increased tumor growth inhibition in subjects relative to treatment with only the KRas G12C inhibitor. In one embodiment, the therapeutically effective amount of the combination of a CDK 4/6 inhibitor of the invention or a pharmaceutically composition thereof and a KRas G12C inhibitor compound of the invention, or a pharmaceutically acceptable salt or a pharmaceutical composition thereof, results in an improvement in the duration of stable disease in subjects compared to treatment with only the KRas G12C inhibitor. In the present invention, the KRas G12C inhibitor is selected from the group consisting of: 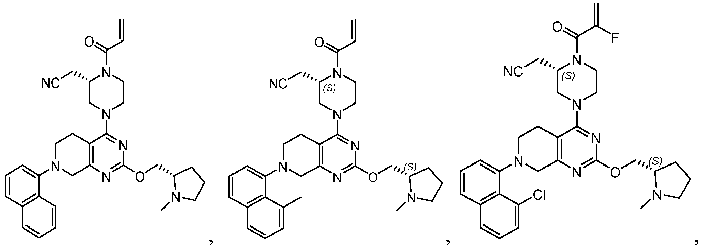 and 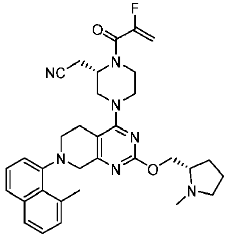 or pharmaceutically acceptable salts thereof. In the present invention, the CDK 4/6 inhibitor is selected from palbociclib or abemaciclib. In one embodiment, the therapeutic combination comprises therapeutically effective amounts of Example No. 234 and palbociclib. In one embodiment, the therapeutic combination comprises therapeutically effective amounts of Example No. 234 and abemaciclib. In one embodiment, the therapeutic combination comprises therapeutically effective amounts of Example No. 359 and palbociclib. In one embodiment, the therapeutic combination comprises therapeutically effective amounts of Example No. 359 and abemaciclib. In one embodiment, the therapeutic combination comprises therapeutically effective amounts of Example No. 478 and palbociclib. In one embodiment, the therapeutic combination comprises therapeutically effective amounts of Example No. 478 and abemaciclib. In one embodiment, the therapeutic combination comprises therapeutically effective amounts of Example No. 507 and palbociclib. In one embodiment, the therapeutic combination comprises therapeutically effective amounts of Example No. 507 and abemaciclib.

In another embodiment, the CDK 4/6 inhibitor or a pharmaceutically composition thereof is administered in combination with the KRas G12C inhibitor, or a pharmaceutically acceptable salt or a pharmaceutically composition thereof once disease progression has been observed for KRas G12C monotherapy, in which the combination therapy results in enhanced clinical benefit for the patient by increasing OS, PFS, tumor regression, tumor growth inhibition or the duration of stable disease in the patient. In the present invention, the KRas G12C inhibitor is selected from the group consisting of: 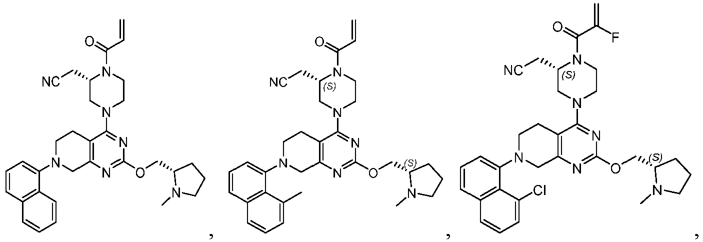 and 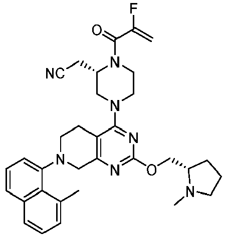 or pharmaceutically acceptable salts thereof. In teh present invention, the CDK 4/6 inhibitor is selected from palbociclib or abemaciclib. In one embodiment, the therapeutic combination comprises therapeutically effective amounts of Example No. 234 and palbociclib. In one embodiment, the therapeutic combination comprises therapeutically effective amounts of Example No. 234 and abemaciclib. In one embodiment, the therapeutic combination comprises therapeutically effective amounts of Example No. 359 and palbociclib. In one embodiment, the therapeutic combination comprises therapeutically effective amounts of Example No. 359 and abemaciclib. In one embodiment, the therapeutic combination comprises therapeutically effective amounts of Example No. 478 and palbociclib. In one embodiment, the therapeutic combination comprises therapeutically effective amounts of Example No. 478 and abemaciclib. In one embodiment, the therapeutic combination comprises therapeutically effective amounts of Example No. 507 and palbociclib. In one embodiment, the therapeutic combination comprises therapeutically effective amounts of Example No. 507 and abemaciclib.

The compositions provided herein may be used for the treatment of a wide variety of cancers including tumors such as lung, colorectal, pancreas, prostate, breast, brain, skin, cervical carcinomas, testicular carcinomas, etc. More particularly, cancers that may be treated by the compositions of the invention include, but are not limited to, tumor types such as astrocytic, breast, cervical, colorectal, endometrial, esophageal, gastric, head and neck, hepatocellular, laryngeal, lung, oral, ovarian, prostate and thyroid carcinomas and sarcomas. More specifically, these compounds can be used to treat: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma. In certain embodiments, the cancer is non-small cell lung cancer.

Also provided herein is a combination of a CDK 4/6 inhibitor or a pharmaceutically composition thereof and a KRas G12C inhibitor compound, or a pharmaceutically acceptable salt or a pharmaceutical composition thereof for use in a method for treating cancer in a subject in need thereof, the method comprising (a) determining that cancer is associated with a KRas G12C mutation (e.g., a KRas G12C-associated cancer) (e.g., as determined using a regulatory agency-approved, e.g., FDA-approved, assay or kit); and (b) administering to the patient a therapeutically effective amount of the combination of a CDK 4/6 inhibitor or a pharmaceutically composition thereof and a KRas G12C inhibitor compound, or a pharmaceutically acceptable salt or a pharmaceutical composition thereof, wherein the CDK 4/6 inhibitor is abemaciclib or palbociclib, wherein the KRas G12C inhibitor is selected from the group consisting of: 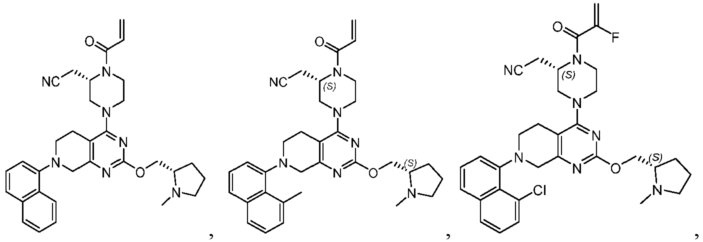 and 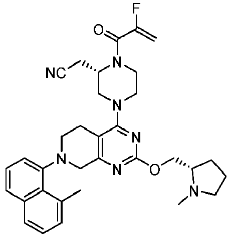 or pharmaceutically acceptable salts thereof, and wherein the CDK 4/6 inhibitor synergistically increases the sensitivity of the KRas G12C-associated cancer to the KRas G12C inhibitor. In one embodiment, the therapeutic combination comprises therapeutically effective amounts of Example No. 234 and palbociclib. In one embodiment, the therapeutic combination comprises therapeutically effective amounts of Example No. 234 and abemaciclib. In one embodiment, the therapeutic combination comprises therapeutically effective amounts of Example No. 359 and palbociclib. In one embodiment, the therapeutic combination comprises therapeutically effective amounts of Example No. 359 and abemaciclib. In one embodiment, the therapeutic combination comprises therapeutically effective amounts of Example No. 478 and palbociclib. In one embodiment, the therapeutic combination comprises therapeutically effective amounts of Example No. 478 and abemaciclib. In one embodiment, the therapeutic combination comprises therapeutically effective amounts of Example No. 507 and palbociclib. In one embodiment, the therapeutic combination comprises therapeutically effective amounts of Example No. 507 and abemaciclib.

In one embodiment, the KRas G12C inhibitor of the invention is administered as a capsule during the period of time. In one embodiment, a tablet or capsule formulation of a compound of Formula I comprises about 10 mg to about 100 mg (e.g., about 10 mg to about 95 mg, about 10 mg to about 90 mg, about 10 mg to about 85 mg, about 10 mg to about 80 mg, about 10 mg to about 75 mg, about 10 mg to about 70 mg, about 10 mg to about 65 mg, about 10 mg to about 60 mg, about 10 mg to about 55 mg, about 10 mg to about 50 mg, about 10 mg to about 45 mg, about 10 mg to about 40 mg, about 10 mg to about 35 mg, about 10 mg to about 30 mg, about 10 mg to about 25 mg, about 10 mg to about 20 mg, about 10 mg to about 15 mg, about 15 mg to about 100 mg, about 15 mg to about 95 mg, about 15 mg to about 90 mg, about 15 mg to about 85 mg, about 15 mg to about 80 mg, about 15 mg to about 75 mg, about 15 mg to about 70 mg, about 15 mg to about 65 mg, about 15 mg to about 60 mg, about 15 mg to about 55 mg, about 15 mg to about 50 mg, about 15 mg to about 45 mg, about 15 mg to about 40 mg, about 15 mg to about 35 mg, about 15 mg to about 30 mg, about 15 mg to about 25 mg, about 15 mg to about 20 mg, about 20 mg to about 100 mg, about 20 mg to about 95 mg, about 20 mg to about 90 mg, about 20 mg to about 85 mg, about 20 mg to about 80 mg, about 20 mg to about 75 mg, about 20 mg to about 70 mg, about 20 mg to about 65 mg, about 20 mg to about 60 mg, about 20 mg to about 55 mg, about 20 mg to about 50 mg, about 20 mg to about 45 mg, about 20 mg to about 40 mg, about 20 mg to about 35 mg, about 20 mg to about 30 mg, about 20 mg to about 25 mg, about 25 mg to about 100 mg, about 25 mg to about 95 mg, about 25 mg to about 90 mg, about 25 mg to about 85 mg, about 25 mg to about 80 mg, about 25 mg to about 75 mg, about 25 mg to about 70 mg, about 25 mg to about 65 mg, about 25 mg to about 60 mg, about 25 mg to about 55 mg, about 25 mg to about 50 mg, about 25 mg to about 45 mg, about 25 mg to about 40 mg, about 25 mg to about 35 mg, about 25 mg to about 30 mg, about 30 mg to about 100 mg, about 30 mg to about 95 mg, about 30 mg to about 90 mg, about 30 mg to about 85 mg, about 30 mg to about 80 mg, about 30 mg to about 75 mg, about 30 mg to about 70 mg, about 30 mg to about 65 mg, about 30 mg to about 60 mg, about 30 mg to about 55 mg, about 30 mg to about 50 mg, about 30 mg to about 45 mg, about 30 mg to about 40 mg, about 30 mg to about 35 mg, about 35 mg to about 100 mg, about 35 mg to about 95 mg, about 35 mg to about 90 mg, about 35 mg to about 85 mg, about 35 mg to about 80 mg, about 35 mg to about 75 mg, about 35 mg to about 70 mg, about 35 mg to about 65 mg, about 35 mg to about 60 mg, about 35 mg to about 55 mg, about 35 mg to about 50 mg, about 35 mg to about 45 mg, about 35 mg to about 40 mg, about 40 mg to about 100 mg, about 40 mg to about 95 mg, about 40 mg to about 90 mg, about 40 mg to about 85 mg, about 40 mg to about 80 mg, about 40 mg to about 75 mg, about 40 mg to about 70 mg, about 40 mg to about 65 mg, about 40 mg to about 60 mg, about 40 mg to about 55 mg, about 40 mg to about 50 mg, about 40 mg to about 45 mg, about 45 mg to about 100 mg, about 45 mg to about 95 mg, about 45 mg to about 90 mg, about 45 mg to about 85 mg, about 45 mg to about 80 mg, about 45 mg to about 75 mg, about 45 mg to about 70 mg, about 45 mg to about 65 mg, about 45 mg to about 60 mg, about 45 mg to about 55 mg, about 45 mg to about 50 mg, about 50 mg to about 100 mg, about 50 mg to about 95 mg, about 50 mg to about 90 mg, about 50 mg to about 85 mg, about 50 mg to about 80 mg, about 50 mg to about 75 mg, about 50 mg to about 70 mg, about 50 mg to about 65 mg, about 50 mg to about 60 mg, about 50 mg to about 55 mg, about 55 mg to about 100 mg, about 55 mg to about 95 mg, about 55 mg to about 90 mg, about 55 mg to about 85 mg, about 55 mg to about 80 mg, about 55 mg to about 75 mg, about 55 mg to about 70 mg, about 55 mg to about 65 mg, about 55 mg to about 60 mg, about 60 mg to about 100 mg, about 60 mg to about 95 mg, about 60 mg to about 90 mg, about 60 mg to about 85 mg, about 60 mg to about 80 mg, about 60 mg to about 75 mg, about 60 mg to about 70 mg, about 60 mg to about 65 mg, about 65 mg to about 100 mg, about 65 mg to about 95 mg, about 65 mg to about 90 mg, about 65 mg to about 85 mg, about 65 mg to about 80 mg, about 65 mg to about 75 mg, about 65 mg to about 70 mg, about 70 mg to about 100 mg, about 70 mg to about 95 mg, about 70 mg to about 90 mg, about 70 mg to about 85 mg, about 70 mg to about 80 mg, about 70 mg to about 75 mg, about 75 mg to about 100 mg, about 75 mg to about 95 mg, about 75 mg to about 90 mg, about 75 mg to about 85 mg, about 75 mg to about 80 mg, about 80 mg to about 100 mg, about 80 mg to about 95 mg, about 80 mg to about 90 mg, about 80 mg to about 85 mg, about 85 mg to about 100 mg, about 85 mg to about 95 mg, about 85 mg to about 90 mg, about 90 mg to about 100 mg, about 90 mg to about 95 mg, about 95 mg to about 100 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg) of the KRas G12C inhibitor of the invention. In one embodiment, the KRas G12C inhibitor of the invention is orally administered once a day (QD) on a daily basis during a period of time. In one embodiment, the KRas G12C inhibitor of the invention is orally administered twice a day (BID) on a daily basis during a period of time. In one embodiment, the KRas G12C inhibitor of the invention is orally administered in the amount of about 20 mg to about 500 mg (e.g., about 20 mg to about 480 mg, about 20 mg to about 460 mg, about 20 mg to about 440 mg, about 20 mg to about 420 mg, about 20 mg to about 400 mg, about 20 mg to about 380 mg, about 20 mg to about 360 mg, about 20 mg to about 340 mg, about 20 mg to about 320 mg, about 20 mg to about 300 mg, about 20 mg to about 280 mg, about 20 mg to about 260 mg, about 20 mg to about 240 mg, about 20 mg to about 220 mg, about 20 mg to about 200 mg, about 20 mg to about 180 mg, about 20 mg to about 160 mg, about 20 mg to about 140 mg, about 20 mg to about 120 mg, about 20 mg to about 100 mg, about 20 mg to about 80 mg, about 20 mg to about 60 mg, about 20 mg to about 40 mg, about 40 mg to about 500 mg, about 40 mg to about 480 mg, about 40 mg to about 460 mg, about 40 mg to about 440 mg, about 40 mg to about 420 mg, about 40 mg to about 400 mg, about 40 mg to about 380 mg, about 40 mg to about 360 mg, about 40 mg to about 340 mg, about 40 mg to about 320 mg, about 40 mg to about 300 mg, about 40 mg to about 280 mg, about 40 mg to about 260 mg, about 40 mg to about 240 mg, about 40 mg to about 220 mg, about 40 mg to about 200 mg, about 40 mg to about 180 mg, about 40 mg to about 160 mg, about 40 mg to about 140 mg, about 40 mg to about 120 mg, about 40 mg to about 100 mg, about 40 mg to about 80 mg, about 40 mg to about 60 mg, about 60 mg to about 500 mg, about 60 mg to about 480 mg, about 60 mg to about 460 mg, about 60 mg to about 440 mg, about 60 mg to about 420 mg, about 60 mg to about 400 mg, about 60 mg to about 380 mg, about 60 mg to about 360 mg, about 60 mg to about 340 mg, about 60 mg to about 320 mg, about 60 mg to about 300 mg, about 60 mg to about 280 mg, about 60 mg to about 260 mg, about 60 mg to about 240 mg, about 60 mg to about 220 mg, about 60 mg to about 200 mg, about 60 mg to about 180 mg, about 60 mg to about 160 mg, about 60 mg to about 140 mg, about 60 mg to about 120 mg, about 60 mg to about 100 mg, about 60 mg to about 80 mg, about 80 mg to about 500 mg, about 80 mg to about 480 mg, about 80 mg to about 460 mg, about 80 mg to about 440 mg, about 80 mg to about 420 mg, about 80 mg to about 400 mg, about 80 mg to about 380 mg, about 80 mg to about 360 mg, about 80 mg to about 340 mg, about 80 mg to about 320 mg, about 80 mg to about 300 mg, about 80 mg to about 280 mg, about 80 mg to about 260 mg, about 80 mg to about 240 mg, about 80 mg to about 220 mg, about 80 mg to about 200 mg, about 80 mg to about 180 mg, about 80 mg to about 160 mg, about 80 mg to about 140 mg, about 80 mg to about 120 mg, about 80 mg to about 100 mg, about 100 mg to about 500 mg, about 100 mg to about 480 mg, about 100 mg to about 460 mg, about 100 mg to about 440 mg, about 100 mg to about 420 mg, about 100 mg to about 400 mg, about 100 mg to about 380 mg, about 100 mg to about 360 mg, about 100 mg to about 340 mg, about 100 mg to about 320 mg, about 100 mg to about 300 mg, about 100 mg to about 280 mg, about 100 mg to about 260 mg, about 100 mg to about 240 mg, about 100 mg to about 220 mg, about 100 mg to about 200 mg, about 100 mg to about 180 mg, about 100 mg to about 160 mg, about 100 mg to about 140 mg, about 100 mg to about 120 mg, about 120 mg to about 500 mg, about 120 mg to about 480 mg, about 120 mg to about 460 mg, about 120 mg to about 440 mg, about 120 mg to about 420 mg, about 120 mg to about 400 mg, about 120 mg to about 380 mg, about 120 mg to about 360 mg, about 120 mg to about 340 mg, about 120 mg to about 320 mg, about 120 mg to about 300 mg, about 120 mg to about 280 mg, about 120 mg to about 260 mg, about 120 mg to about 240 mg, about 120 mg to about 220 mg, about 120 mg to about 200 mg, about 120 mg to about 180 mg, about 120 mg to about 160 mg, about 120 mg to about 140 mg, about 140 mg to about 500 mg, about 140 mg to about 480 mg, about 140 mg to about 460 mg, about 140 mg to about 440 mg, about 140 mg to about 420 mg, about 140 mg to about 400 mg, about 140 mg to about 380 mg, about 140 mg to about 360 mg, about 140 mg to about 340 mg, about 140 mg to about 320 mg, about 140 mg to about 300 mg, about 140 mg to about 280 mg, about 140 mg to about 260 mg, about 140 mg to about 240 mg, about 140 mg to about 220 mg, about 140 mg to about 200 mg, about 140 mg to about 180 mg, about 140 mg to about 160 mg, about 160 mg to about 500 mg, about 160 mg to about 480 mg, about 160 mg to about 460 mg, about 160 mg to about 440 mg, about 160 mg to about 420 mg, about 160 mg to about 400 mg, about 160 mg to about 380 mg, about 160 mg to about 360 mg, about 160 mg to about 340 mg, about 160 mg to about 320 mg, about 160 mg to about 300 mg, about 160 mg to about 280 mg, about 160 mg to about 260 mg, about 160 mg to about 240 mg, about 160 mg to about 220 mg, about 160 mg to about 200 mg, about 160 mg to about 180 mg, about 180 mg to about 500 mg, about 180 mg to about 480 mg, about 180 mg to about 460 mg, about 180 mg to about 440 mg, about 180 mg to about 420 mg, about 180 mg to about 400 mg, about 180 mg to about 380 mg, about 180 mg to about 360 mg, about 180 mg to about 340 mg, about 180 mg to about 320 mg, about 180 mg to about 300 mg, about 180 mg to about 280 mg, about 180 mg to about 260 mg, about 180 mg to about 240 mg, about 180 mg to about 220 mg, about 180 mg to about 200 mg, about 200 mg to about 500 mg, about 200 mg to about 480 mg, about 200 mg to about 460 mg, about 200 mg to about 440 mg, about 200 mg to about 420 mg, about 200 mg to about 400 mg, about 200 mg to about 380 mg, about 200 mg to about 360 mg, about 200 mg to about 340 mg, about 200 mg to about 320 mg, about 200 mg to about 300 mg, about 200 mg to about 280 mg, about 200 mg to about 260 mg, about 200 mg to about 240 mg, about 200 mg to about 220 mg, about 220 mg to about 500 mg, about 220 mg to about 480 mg, about 220 mg to about 460 mg, about 220 mg to about 440 mg, about 220 mg to about 420 mg, about 220 mg to about 400 mg, about 220 mg to about 380 mg, about 220 mg to about 360 mg, about 220 mg to about 340 mg, about 220 mg to about 320 mg, about 220 mg to about 300 mg, about 220 mg to about 280 mg, about 220 mg to about 260 mg, about 220 mg to about 240 mg, about 240 mg to about 500 mg, about 240 mg to about 480 mg, about 240 mg to about 460 mg, about 240 mg to about 440 mg, about 240 mg to about 420 mg, about 240 mg to about 400 mg, about 240 mg to about 380 mg, about 240 mg to about 360 mg, about 240 mg to about 340 mg, about 240 mg to about 320 mg, about 240 mg to about 300 mg, about 240 mg to about 280 mg, about 240 mg to about 260 mg, about 260 mg to about 500 mg, about 260 mg to about 480 mg, about 260 mg to about 460 mg, about 260 mg to about 440 mg, about 260 mg to about 420 mg, about 260 mg to about 400 mg, about 260 mg to about 380 mg, about 260 mg to about 360 mg, about 260 mg to about 340 mg, about 260 mg to about 320 mg, about 260 mg to about 300 mg, about 260 mg to about 280 mg, about 280 mg to about 500 mg, about 280 mg to about 480 mg, about 280 mg to about 460 mg, about 280 mg to about 440 mg, about 280 mg to about 420 mg, about 280 mg to about 400 mg, about 280 mg to about 380 mg, about 280 mg to about 360 mg, about 280 mg to about 340 mg, about 280 mg to about 320 mg, about 280 mg to about 300 mg, about 300 mg to about 500 mg, about 300 mg to about 480 mg, about 300 mg to about 460 mg, about 300 mg to about 440 mg, about 300 mg to about 420 mg, about 300 mg to about 400 mg, about 300 mg to about 380 mg, about 300 mg to about 360 mg, about 300 mg to about 340 mg, about 300 mg to about 320 mg, about 320 mg to about 500 mg, about 320 mg to about 480 mg, about 320 mg to about 460 mg, about 320 mg to about 440 mg, about 320 mg to about 420 mg, about 320 mg to about 400 mg, about 320 mg to about 380 mg, about 320 mg to about 360 mg, about 320 mg to about 340 mg, about 340 mg to about 500 mg, about 340 mg to about 480 mg, about 340 mg to about 460 mg, about 340 mg to about 440 mg, about 340 mg to about 420 mg, about 340 mg to about 400 mg, about 340 mg to about 380 mg, about 340 mg to about 360 mg, about 360 mg to about 500 mg, about 360 mg to about 480 mg, about 360 mg to about 460 mg, about 360 mg to about 440 mg, about 360 mg to about 420 mg, about 360 mg to about 400 mg, about 360 mg to about 380 mg, about 380 mg to about 500 mg, about 380 mg to about 480 mg, about 380 mg to about 460 mg, about 380 mg to about 440 mg, about 380 mg to about 420 mg, about 380 mg to about 400 mg, about 400 mg to about 500 mg, about 400 mg to about 480 mg, about 400 mg to about 460 mg, about 400 mg to about 440 mg, about 400 mg to about 420 mg, about 420 mg to about 500 mg, about 420 mg to about 480 mg, about 420 mg to about 460 mg, about 420 mg to about 440 mg, about 440 mg to about 500 mg, about 440 mg to about 480 mg, about 440 mg to about 460 mg, about 460 mg to about 500 mg, about 460 mg to about 480 mg, about 480 mg to about 500 mg, about 25, about 50, about 75, about 100, about 150, about 200, about 250, about 300, about 350, about 400, about 450, or about 500 mg), during a period of time.

In one embodiment, the combination therapy comprises oral administration of the KRas G12C inhibitor of the invention once or twice a day on a daily basis (during a period of time), e.g., in an amount of about 10 mg to about 400 mg (e.g., about 10 mg to about 380 mg, about 10 mg to about 360 mg, about 10 mg to about 340 mg, about 10 mg to about 320 mg, about 10 mg to about 300 mg, about 10 mg to about 280 mg, about 10 mg to about 260 mg, about 10 mg to about 240 mg, about 10 mg to about 220 mg, about 10 mg to about 200 mg, about 10 mg to about 180 mg, about 10 mg to about 160 mg, about 10 mg to about 140 mg, about 10 mg to about 120 mg, about 10 mg to about 100 mg, about 10 mg to about 80 mg, about 10 mg to about 60 mg, about 10 mg to about 40 mg, about 10 mg to about 20 mg, about 20 mg to about 400 mg, about 20 mg to about 380 mg, about 20 mg to about 360 mg, about 20 mg to about 340 mg, about 20 mg to about 320 mg, about 20 mg to about 300 mg, about 20 mg to about 280 mg, about 20 mg to about 260 mg, about 20 mg to about 240 mg, about 20 mg to about 220 mg, about 20 mg to about 200 mg, about 20 mg to about 180 mg, about 20 mg to about 160 mg, about 20 mg to about 140 mg, about 20 mg to about 120 mg, about 20 mg to about 100 mg, about 20 mg to about 80 mg, about 20 mg to about 60 mg, about 20 mg to about 40 mg, about 40 mg to about 400 mg, about 40 mg to about 380 mg, about 40 mg to about 360 mg, about 40 mg to about 340 mg, about 40 mg to about 320 mg, about 40 mg to about 300 mg, about 40 mg to about 280 mg, about 40 mg to about 260 mg, about 40 mg to about 240 mg, about 40 mg to about 220 mg, about 40 mg to about 200 mg, about 40 mg to about 180 mg, about 40 mg to about 160 mg, about 40 mg to about 140 mg, about 40 mg to about 120 mg, about 40 mg to about 100 mg, about 40 mg to about 80 mg, about 40 mg to about 60 mg, about 60 mg to about 400 mg, about 60 mg to about 380 mg, about 60 mg to about 360 mg, about 60 mg to about 340 mg, about 60 mg to about 320 mg, about 60 mg to about 300 mg, about 60 mg to about 280 mg, about 60 mg to about 260 mg, about 60 mg to about 240 mg, about 60 mg to about 220 mg, about 60 mg to about 200 mg, about 60 mg to about 180 mg, about 60 mg to about 160 mg, about 60 mg to about 140 mg, about 60 mg to about 120 mg, about 60 mg to about 100 mg, about 60 mg to about 80 mg, about 80 mg to about 400 mg, about 80 mg to about 380 mg, about 80 mg to about 360 mg, about 80 mg to about 340 mg, about 80 mg to about 320 mg, about 80 mg to about 300 mg, about 80 mg to about 280 mg, about 80 mg to about 260 mg, about 80 mg to about 240 mg, about 80 mg to about 220 mg, about 80 mg to about 200 mg, about 80 mg to about 180 mg, about 80 mg to about 160 mg, about 80 mg to about 140 mg, about 80 mg to about 120 mg, about 80 mg to about 100 mg, about 100 mg to about 400 mg, about 100 mg to about 380 mg, about 100 mg to about 360 mg, about 100 mg to about 340 mg, about 100 mg to about 320 mg, about 100 mg to about 300 mg, about 100 mg to about 280 mg, about 100 mg to about 260 mg, about 100 mg to about 240 mg, about 100 mg to about 220 mg, about 100 mg to about 200 mg, about 100 mg to about 180 mg, about 100 mg to about 160 mg, about 100 mg to about 140 mg, about 100 mg to about 120 mg, about 120 mg to about 400 mg, about 120 mg to about 380 mg, about 120 mg to about 360 mg, about 120 mg to about 340 mg, about 120 mg to about 320 mg, about 120 mg to about 300 mg, about 120 mg to about 280 mg, about 120 mg to about 260 mg, about 120 mg to about 240 mg, about 120 mg to about 220 mg, about 120 mg to about 200 mg, about 120 mg to about 180 mg, about 120 mg to about 160 mg, about 120 mg to about 140 mg, about 140 mg to about 400 mg, about 140 mg to about 380 mg, about 140 mg to about 360 mg, about 140 mg to about 340 mg, about 140 mg to about 320 mg, about 140 mg to about 300 mg, about 140 mg to about 280 mg, about 140 mg to about 260 mg, about 140 mg to about 240 mg, about 140 mg to about 220 mg, about 140 mg to about 200 mg, about 140 mg to about 180 mg, about 140 mg to about 160 mg, about 160 mg to about 400 mg, about 160 mg to about 380 mg, about 160 mg to about 360 mg, about 160 mg to about 360 mg, about 160 mg to about 340 mg, about 160 mg to about 320 mg, about 160 mg to about 300 mg, about 160 mg to about 280 mg, about 160 mg to about 260 mg, about 160 mg to about 240 mg, about 160 mg to about 220 mg, about 160 mg to about 200 mg, about 160 mg to about 180 mg, about 180 mg to about 400 mg, about 180 mg to about 380 mg, about 180 mg to about 360 mg, about 180 mg to about 340 mg, about 180 mg to about 320 mg, about 180 mg to about 300 mg, about 180 mg to about 280 mg, about 180 mg to about 260 mg, about 180 mg to about 240 mg, about 180 mg to about 220 mg, about 180 mg to about 200 mg, about 200 mg to about 400 mg, about 200 mg to about 380 mg, about 200 mg to about 360 mg, about 200 mg to about 340 mg, about 200 mg to about 320 mg, about 200 mg to about 300 mg, about 200 mg to about 280 mg, about 200 mg to about 260 mg, about 200 mg to about 240 mg, about 200 mg to about 220 mg, about 220 mg to about 400 mg, about 220 mg to about 380 mg, about 220 mg to about 360 mg, about 220 mg to about 340 mg, about 220 mg to about 320 mg, about 220 mg to about 300 mg, about 220 mg to about 280 mg, about 220 mg to about 260 mg, about 220 mg to about 240 mg, about 240 mg to about 400 mg, about 240 mg to about 380 mg, about 240 mg to about 360 mg, about 240 mg to about 340 mg, about 240 mg to about 320 mg, about 240 mg to about 300 mg, about 240 mg to about 280 mg, about 240 mg to about 260 mg, about 260 mg to about 400 mg, about 260 mg to about 380 mg, about 260 mg to about 360 mg, about 260 mg to about 340 mg, about 260 mg to about 320 mg, about 260 mg to about 300 mg, about 260 mg to about 280 mg, about 280 mg to about 400 mg, about 280 mg to about 380 mg, about 280 mg to about 360 mg, about 280 mg to about 340 mg, about 280 mg to about 320 mg, about 280 mg to about 300 mg, about 300 mg to about 400 mg, about 300 mg to about 380 mg, about 300 mg to about 360 mg, about 300 mg to about 340 mg, about 300 mg to about 320 mg, about 320 mg to about 400 mg, about 320 mg to about 380 mg, about 320 mg to about 360 mg, about 340 mg to about 360 mg, about 340 mg to about 400 mg, about 340 mg to about 380 mg, about 340 mg to about 360 mg, about 360 mg to about 400 mg, about 360 mg to about 380 mg, about 380 mg to about 400 mg, about 100 mg, about 200 mg, about 300 mg, or about 400 mg), and oral administration of the CDK 4/6 inhibitor of the invention which is administered, for example once a day on a daily basis (during a period of time). In one embodiment, the KRAS inhibitor is orally administered once daily. In one embodiment, the KRAS inhibitor is orally administered twice daily.

One skilled in the art will recognize that, both in vivo and in vitro trials using suitable, known and generally accepted cell and/or animal models are predictive of the ability of a test compound of the combination or the combination to treat or prevent a given disorder.

One skilled in the art will further recognize that human clinical trials including first-inhuman, dose ranging and efficacy trials, in healthy patients and/or those suffering from a given disorder, may be completed according to methods well known in the clinical and medical arts.

### SYNERGY

In one embodiment, the addition of a CDK 4/6 inhibitor of the invention or a pharmaceutically composition thereof synergistically increases the activity of KRas G12C inhibitor compound of the invention, or a pharmaceutically acceptable salt or a pharmaceutically composition thereof against cancer or cancer cell lines expressing KRas G12C. Any method for determining whether two compounds exhibit synergy may be used for determining the synergistic effect of the combination.

Several mathematical models have been developed to determine whether two compounds act synergistically, i.e., beyond a mere additive effect. For instance, Loewe Additivity (Loewe (1928) Physiol. 27: 47-187), Bliss Independence (Bliss (1939) Ann. Appl. Biol. 26: 585-615), Highest Single Agent, ZIP (Yadav et al (2015) Comput Struct Biotech J 13: 504-513) and other models (Chou & Talalay (1984) Adv Enzyme Regul 22: 27-55. #6382953; and Greco et al. (1995) Pharmacol Rev 47(2): 331-85. #7568331) are well known models in the pharmaceutical industry and may be used to calculate a "synergy score" that indicates whether synergy was detected and the magnitude of such synergy. Combining these synergy scores produces a composite synergy score which may be used to evaluate and characterize the KRas G12C inhibitor compounds of the invention in combination with a CDK 4/6 inhibitor of the invention.

In general, the mathematical models use data obtained from single agent values to determine the predicted additive effect of the combination which is compared to the observed effect for the combination. If the observed effect is greater than the predicted effect, the combination is deemed to be synergistic. For example, the Bliss independence model compares the observed combination response *(Y_{O})* with the predicted combination response *(Y_{P}),* which was obtained based on the assumption that there is no effect from drug-drug interactions. Typically, the combination effect is declared synergistic if *Y_{O}* is greater than *Y_{P}.*

In some embodiments, "synergistic effect" as used herein refers to combination of a KRAS inhibitor or a pharmaceutically acceptable salt thereof, and a CDK 4/6 inhibitor producing an effect, for example, any of the beneficial or desired results including clinical results or endpoints as described herein, which is greater than the sum of the effect observed when the KRas G12C inhibitor of the invention or a pharmaceutically acceptable salt thereof and a CDK 4/6 inhibitor of the invention are administered alone. In the present invention, the KRas G12C inhibitor is selected from the group consisting of: 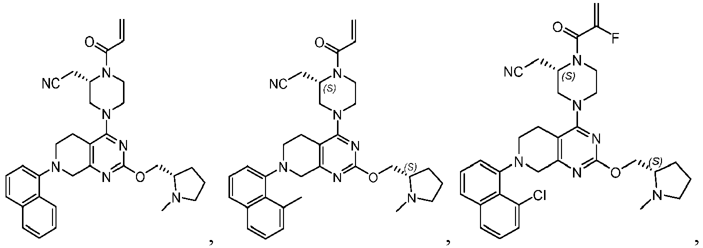 and 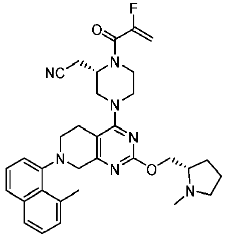 or pharmaceutically acceptable salts thereof. In the present invention, the CDK 4/6 inhibitor is selected from palbociclib or abemaciclib. In one embodiment, the therapeutic combination comprises therapeutically effective amounts of Example No. 234 and palbociclib. In one embodiment, the therapeutic combination comprises therapeutically effective amounts of Example No. 234 and abemaciclib. In one embodiment, the therapeutic combination comprises therapeutically effective amounts of Example No. 359 and palbociclib. In one embodiment, the therapeutic combination comprises therapeutically effective amounts of Example No. 359 and abemaciclib. In one embodiment, the therapeutic combination comprises therapeutically effective amounts of Example No. 478 and palbociclib. In one embodiment, the therapeutic combination comprises therapeutically effective amounts of Example No. 478 and abemaciclib. In one embodiment, the therapeutic combination comprises therapeutically effective amounts of Example No. 507 and palbociclib. In one embodiment, the therapeutic combination comprises therapeutically effective amounts of Example No. 507 and abemaciclib

In some embodiments, the compositions provided herein can result in a 1% to 99% (e.g., 1% to 98%, 1% to 95%, 1% to 90%, 1 to 85%, 1 to 80%, 1% to 75%, 1% to 70%, 1% to 65%, 1% to 60%, 1% to 55%, 1% to 50%, 1% to 45%, 1% to 40%, 1% to 35%, 1% to 30%, 1% to 25%, 1% to 20%, 1% to 15%, 1% to 10%, 1% to 5%, 2% to 99%, 2% to 90%, 2% to 85%, 2% to 80%, 2% to 75%, 2% to 70%, 2% to 65%, 2% to 60%, 2% to 55%, 2% to 50%, 2% to 45%, 2% to 40%, 2% to 35%, 2% to 30%, 2% to 25%, 2% to 20%, 2% to 15%, 2% to 10%, 2% to 5%, 4% to 99%, 4% to 95%, 4% to 90%, 4% to 85%, 4% to 80%, 4% to 75%, 4% to 70%, 4% to 65%, 4% to 60%, 4% to 55%, 4% to 50%, 4% to 45%, 4% to 40%, 4% to 35%, 4% to 30%, 4% to 25%, 4% to 20%, 4% to 15%, 4% to 10%, 6% to 99%, 6% to 95%, 6% to 90%, 6% to 85%, 6% to 80%, 6% to 75%, 6% to 70%, 6% to 65%, 6% to 60%, 6% to 55%, 6% to 50%, 6% to 45%, 6% to 40%, 6% to 35%, 6% to 30%, 6% to 25%, 6% to 20%, 6% to 15%, 6% to 10%, 8% to 99%, 8% to 95%, 8% to 90%, 8% to 85%, 8% to 80%, 8% to 75%, 8% to 70%, 8% to 65%, 8% to 60%, 8% to 55%, 8% to 50%, 8% to 45%, 8% to 40%, 8% to 35%, 8% to 30%, 8% to 25%, 8% to 20%, 8% to 15%, 10% to 99%, 10% to 95%, 10% to 90%, 10% to 85%, 10% to 80%, 10% to 75%, 10% to 70%, 10% to 65%, 10% to 60%, 10% to 55%, 10% to 50%, 10% to 45%, 10% to 40%, 10% to 35%, 10% to 30%, 10% to 25%, 10% to 20%, 10% to 15%, 15% to 99%, 15% to 95%, 15% to 90%, 15% to 85%, 15% to 80%, 15% to 75%, 15% to 70%, 15% to 65%, 15% to 60%, 15% to 55%, 15% to 50%, 15% to 55%, 15% to 50%, 15% to 45%, 15% to 40%, 15% to 35%, 15% to 30%, 15% to 25%, 15% to 20%, 20% to 99%, 20% to 95%, 20% to 90%, 20% to 85%, 20% to 80%, 20% to 75%, 20% to 70%, 20% to 65%, 20% to 60%, 20% to 55%, 20% to 50%, 20% to 45%, 20% to 40%, 20% to 35%, 20% to 30%, 20% to 25%, 25% to 99%, 25% to 95%, 25% to 90%, 25% to 85%, 25% to 80%, 25% to 75%, 25% to 70%, 25% to 65%, 25% to 60%, 25% to 55%, 25% to 50%, 25% to 45%, 25% to 40%, 25% to 35%, 25% to 30%, 30% to 99%, 30% to 95%, 30% to 90%, 30% to 85%, 30% to 80%, 30% to 75%, 30% to 70%, 30% to 65%, 30% to 60%, 30% to 55%, 30% to 50%, 30% to 45%, 30% to 40%, 30% to 35%, 35% to 99%, 35% to 95%, 35% to 90%, 35% to 85%, 35% to 80%, 35% to 75%, 35% to 70%, 35% to 65%, 35% to 60%, 35% to 55%, 35% to 50%, 35% to 45%, 35% to 40%, 40% to 99%, 40% to 95%, 40% to 90%, 40% to 85%, 40% to 80%, 40% to 75%, 40% to 70%, 40% to 65%, 40% to 60%, 40% to 55%, 40% to 60%, 40% to 55%, 40% to 50%, 40% to 45%, 45% to 99%, 45% to 95%, 45% to 95%, 45% to 90%, 45% to 85%, 45% to 80%, 45% to 75%, 45% to 70%, 45% to 65%, 45% to 60%, 45% to 55%, 45% to 50%, 50% to 99%, 50% to 95%, 50% to 90%, 50% to 85%, 50% to 80%, 50% to 75%, 50% to 70%, 50% to 65%, 50% to 60%, 50% to 55%, 55% to 99%, 55% to 95%, 55% to 90%, 55% to 85%, 55% to 80%, 55% to 75%, 55% to 70%, 55% to 65%, 55% to 60%, 60% to 99%, 60% to 95%, 60% to 90%, 60% to 85%, 60% to 80%, 60% to 75%, 60% to 70%, 60% to 65%, 65% to 99%, 60% to 95%, 60% to 90%, 60% to 85%, 60% to 80%, 60% to 75%, 60% to 70%, 60% to 65%, 70% to 99%, 70% to 95%, 70% to 90%, 70% to 85%, 70% to 80%, 70% to 75%, 75% to 99%, 75% to 95%, 75% to 90%, 75% to 85%, 75% to 80%, 80% to 99%, 80% to 95%, 80% to 90%, 80% to 85%, 85% to 99%, 85% to 95%, 85% to 90%, 90% to 99%, 90% to 95%, or 95% to 100%) reduction in the volume of one or more solid tumors in a patient following treatment with the combination therapy for a period of time between 1 day and 2 years (e.g., between 1 day and 22 months, between 1 day and 20 months, between 1 day and 18 months, between 1 day and 16 months, between 1 day and 14 months, between 1 day and 12 months, between 1 day and 10 months, between 1 day and 9 months, between 1 day and 8 months, between 1 day and 7 months, between 1 day and 6 months, between 1 day and 5 months, between 1 day and 4 months, between 1 day and 3 months, between 1 day and 2 months, between 1 day and 1 month, between one week and 2 years, between 1 week and 22 months, between 1 week and 20 months, between 1 week and 18 months, between 1 week and 16 months, between 1 week and 14 months, between 1 week and 12 months, between 1 week and 10 months, between 1 week and 9 months, between 1 week and 8 months, between 1 week and 7 months, between 1 week and 6 months, between 1 week and 5 months, between 1 week and 4 months, between 1 week and 3 months, between 1 week and 2 months, between 1 week and 1 month, between 2 weeks and 2 years, between 2 weeks and 22 months, between 2 weeks and 20 months, between 2 weeks and 18 months, between 2 weeks and 16 months, between 2 weeks and 14 months, between 2 weeks and 12 months, between 2 weeks and 10 months, between 2 weeks and 9 months, between 2 weeks and 8 months, between 2 weeks and 7 months, between 2 weeks and 6 months, between 2 weeks and 5 months, between 2 weeks and 4 months, between 2 weeks and 3 months, between 2 weeks and 2 months, between 2 weeks and 1 month, between 1 month and 2 years, between 1 month and 22 months, between 1 month and 20 months, between 1 month and 18 months, between 1 month and 16 months, between 1 month and 14 months, between 1 month and 12 months, between 1 month and 10 months, between 1 month and 9 months, between 1 month and 8 months, between 1 month and 7 months, between 1 month and 6 months, between 1 month and 6 months, between 1 month and 5 months, between 1 month and 4 months, between 1 month and 3 months, between 1 month and 2 months, between 2 months and 2 years, between 2 months and 22 months, between 2 months and 20 months, between 2 months and 18 months, between 2 months and 16 months, between 2 months and 14 months, between 2 months and 12 months, between 2 months and 10 months, between 2 months and 9 months, between 2 months and 8 months, between 2 months and 7 months, between 2 months and 6 months, or between 2 months and 5 months, between 2 months and 4 months, between 3 months and 2 years, between 3 months and 22 months, between 3 months and 20 months, between 3 months and 18 months, between 3 months and 16 months, between 3 months and 14 months, between 3 months and 12 months, between 3 months and 10 months, between 3 months and 8 months, between 3 months and 6 months, between 4 months and 2 years, between 4 months and 22 months, between 4 months and 20 months, between 4 months and 18 months, between 4 months and 16 months, between 4 months and 14 months, between 4 months and 12 months, between 4 months and 10 months, between 4 months and 8 months, between 4 months and 6 months, between 6 months and 2 years, between 6 months and 22 months, between 6 months and 20 months, between 6 months and 18 months, between 6 months and 16 months, between 6 months and 14 months, between 6 months and 12 months, between 6 months and 10 months, or between 6 months and 8 months) (e.g., as compared to the size of the one or more solid tumors in the patient prior to treatment).

The phrase "time of survival" means the length of time between the identification or diagnosis of cancer (e.g., any of the cancers described herein) in a mammal by a medical professional and the time of death of the mammal (caused by the cancer). Methods of increasing the time of survival in a mammal having a cancer are described herein.

In some embodiments, any of the compositions described herein can result in an increase (e.g., a 1% to 400%, 1% to 380%, 1% to 360%, 1% to 340%, 1% to 320%, 1% to 300%, 1% to 280%, 1% to 260%, 1% to 240%, 1% to 220%, 1% to 200%, 1% to 180%, 1% to 160%, 1% to 140%, 1% to 120%, 1% to 100%, 1% to 95%, 1% to 90%, 1% to 85%, 1% to 80%, 1% to 75%, 1% to 70%, 1% to 65%, 1% to 60%, 1% to 55%, 1% to 50%, 1% to 45%, 1% to 40%, 1% to 35%, 1% to 30%, 1% to 25%, 1% to 20%, 1% to 15%, 1% to 10%, 1% to 5%, 5% to 400%, 5% to 380%, 5% to 360%, 5% to 340%, 5% to 320%, 5% to 300%, 5% to 280%, 5% to 260%, 5% to 240%, 5% to 220%, 5% to 200%, 5% to 180%, 5% to 160%, 5% to 140%, 5% to 120%, 5% to 100%, 5% to 90%, 5% to 80%, 5% to 70%, 5% to 60%, 5% to 50%, 5% to 40%, 5% to 30%, 5% to 20%, 5% to 10%, 10% to 400%, 10% to 380%, 10% to 360%, 10% to 340%, 10% to 320%, 10% to 300%, 10% to 280%, 10% to 260%, 10% to 240%, 10% to 220%, 10% to 200%, 10% to 180%, 10% to 160%, 10% to 140%, 10% to 120%, 10% to 100%, 10% to 90%, 10% to 80%, 10% to 70%, 10% to 60%, 10% to 50%, 10% to 40%, 10% to 30%, 10% to 20%, 20% to 400%, 20% to 380%, 20% to 360%, 20% to 340%, 20% to 320%, 20% to 300%, 20% to 280%, 20% to 260%, 20% to 240%, 20% to 220%, 20% to 200%, 20% to 180%, 20% to 160%, 20% to 140%, 20% to 120%, 20% to 100%, 20% to 90%, 20% to 80%, 20% to 70%, 20% to 60%, 20% to 50%, 20% to 40%, 20% to 30%, 30% to 400%, 30% to 380%, 30% to 360%, 30% to 340%, 30% to 320%, 30% to 300%, 30% to 280%, 30% to 260%, 30% to 240%, 30% to 220%, 30% to 200%, 30% to 180%, 30% to 160%, 30% to 140%, 30% to 120%, 30% to 100%, 30% to 90%, 30% to 80%, 30% to 70%, 30% to 60%, 30% to 50%, 30% to 40%, 40% to 400%, 40% to 380%, 40% to 360%, 40% to 340%, 40% to 320%, 40% to 300%, 40% to 280%, 40% to 260%, 40% to 240%, 40% to 220%, 40% to 200%, 40% to 180%, 40% to 160%, 40% to 140%, 40% to 120%, 40% to 100%, 40% to 90%, 40% to 80%, 40% to 70%, 40% to 60%, 40% to 50%, 50% to 400%, 50% to 380%, 50% to 360%, 50% to 340%, 50% to 320%, 50% to 300%, 50% to 280%, 50% to 260%, 50% to 240%, 50% to 220%, 50% to 200%, 50% to 180%, 50% to 160%, 50% to 140%, 50% to 140%, 50% to 120%, 50% to 100%, 50% to 90%, 50% to 80%, 50% to 70%, 50% to 60%, 60% to 400%, 60% to 380%, 60% to 360%, 60% to 340%, 60% to 320%, 60% to 300%, 60% to 280%, 60% to 260%, 60% to 240%, 60% to 220%, 60% to 200%, 60% to 180%, 60% to 160%, 60% to 140%, 60% to 120%, 60% to 100%, 60% to 90%, 60% to 80%, 60% to 70%, 70% to 400%, 70% to 380%, 70% to 360%, 70% to 340%, 70% to 320%, 70% to 300%, 70% to 280%, 70% to 260%, 70% to 240%, 70% to 220%, 70% to 200%, 70% to 180%, 70% to 160%, 70% to 140%, 70% to 120%, to 100%, 70% to 90%, 70% to 80%, 80% to 400%, 80% to 380%, 80% to 360%, 80% to 340%, 80% to 320%, 80% to 300%, 80% to 280%, 80% to 260%, 80% to 240%, 80% to 220%, 80% to 200%, 80% to 180%, 80% to 160%, 80% to 140%, 80% to 120%, 80% to 100%, 80% to 90%, 90% to 400%, 90% to 380%, 90% to 360%, 90% to 340%, 90% to 320%, 90% to 300%, 90% to 280%, 90% to 260%, 90% to 240%, 90% to 220%, 90% to 200%, 90% to 180%, 90% to 160%, 90% to 140%, 90% to 120%, 90% to 100%, 100% to 400%, 100% to 380%, 100% to 360%, 100% to 340%, 100% to 320%, 100% to 300%, 100% to 280%, 100% to 260%, 100% to 240%, 100% to 220%, 100% to 200%, 100% to 180%, 100% to 160%, 100% to 140%, 100% to 120%, 120% to 400%, 120% to 380%, 120% to 360%, 120% to 340%, 120% to 320%, 120% to 300%, 120% to 280%, 120% to 260%, 120% to 240%, 120% to 220%, 120% to 200%, 120% to 180%, 120% to 160%, 120% to 140%, 140% to 400%, 140% to 380%, 140% to 360%, 140% to 340%, 140% to 320%, 140% to 300%, 140% to 280%, 140% to 260%, 140% to 240%, 140% to 220%, 140% to 200%, 140% to 180%, 140% to 160%, 160% to 400%, 160% to 380%, 160% to 360%, 160% to 340%, 160% to 320%, 160% to 300%, 160% to 280%, 160% to 260%, 160% to 240%, 160% to 220%, 160% to 200%, 160% to 180%, 180% to 400%, 180% to 380%, 180% to 360%, 180% to 340%, 180% to 320%, 180% to 300%, 180% to 280%, 180% to 260%, 180% to 240%, 180% to 220%, 180% to 200%, 200% to 400%, 200% to 380%, 200% to 360%, 200% to 340%, 200% to 320%, 200% to 300%, 200% to 280%, 200% to 260%, 200% to 240%, 200% to 220%, 220% to 400%, 220% to 380%, 220% to 360%, 220% to 340%, 220% to 320%, 220% to 300%, 220% to 280%, 220% to 260%, 220% to 240%, 240% to 400%, 240% to 380%, 240% to 360%, 240% to 340%, 240% to 320%, 240% to 300%, 240% to 280%, 240% to 260%, 260% to 400%, 260% to 380%, 260% to 360%, 260% to 340%, 260% to 320%, 260% to 300%, 260% to 280%, 280% to 400%, 280% to 380%, 280% to 360%, 280% to 340%, 280% to 320%, 280% to 300%, 300% to 400%, 300% to 380%, 300% to 360%, 300% to 340%, or 300% to 320%) in the time of survival of the patient (e.g., as compared to a patient having a similar cancer and administered a different treatment or not receiving a treatment).

In some embodiments of any of the aspects described herein, before treatment with the compositions of the invention, the patient was treated with one or more of a chemotherapy, a targeted anticancer agent, radiation therapy, and surgery, and optionally, the prior treatment was unsuccessful; and/or the patient has been administered surgery and optionally, the surgery was unsuccessful; and/or the patient has been treated with a platinum-based chemotherapeutic agent, and optionally, the patient has been previously determined to be non-responsive to treatment with the platinum-based chemotherapeutic agent; and/or the patient has been treated with a kinase inhibitor, and optionally, the prior treatment with the kinase inhibitor was unsuccessful; and/or the patient was treated with one or more other therapeutic agent(s).

### KITS

The present invention also relates to a kit comprising a CDK 4/6 inhibitor of the invention or a pharmaceutically composition thereof and a KRas G12C inhibitor compound of the invention. Also provided is a kit comprising a CDK 4/6 inhibitor of the invention or a pharmaceutically composition thereof and a KRas G12C inhibitor compound of the invention, or a pharmaceutically acceptable salt or a pharmaceutically composition thereof, for use in treating a hematological cancer.

In a related aspect, the invention provides a kit containing a dose of a CDK 4/6 inhibitor of the invention or a pharmaceutically composition thereof and a dose of a KRas G12C inhibitor compound of the invention in an amount effective to inhibit proliferation of cancer cells, particularly KRas G12C-expressing cancer cells, in a subject. The kit in some cases includes an insert with instructions for administration of the CDK 4/6 inhibitor or a pharmaceutically composition thereof and a KRas G12C inhibitor compound of the invention, or a pharmaceutically acceptable salt or a pharmaceutically composition thereof. The insert may provide a user with one set of instructions for using the CDK 4/6 inhibitor of the invention in combination with a KRas G12C inhibitor compound of the invention.

### EXAMPLE A

### CDK 4/6 Inhibitors Synergistically Increase the Activity of KRas G12C Inhibitors Against Cell Lines Expressing KRas G12C

This Example illustrates that the combination of KRas G12C inhibitor compounds of the invention and a CDK 4/6 inhibitor of the invention synergistically inhibits the growth of tumor cell lines that express KRas G12C.

A panel of 8 lung cancer and 1 colorectal cell lines harboring KRas G12C mutations was assembled to determine whether combining CDK 4/6 inhibitors with KRas G12C inhibitors disclosed herein results in synergistic activity. The collection included NCI-H1373 (ATCC CRL-5866; CDKN2A C72 mutation); NCI-H1792 (ATCC CRL-5895; CDK 4 amplified); NCI-H2030 (ATCC CRL-5985); NCI-H2122 (ATCC CRL-5985; CDKN2A deleted; CDK 6 amplified); HCC1171 (KCLB 71171; CDKN2A deleted); HCC44 (DSMZ ACC-534); LU99 (RCB1900; CDKN2A deleted); SW1573 (ATCC CRL-2170; CDKN2A deleted) and SW837 (ATCC CCL-235).

Assays for determining the synergy score for the pairwise combinations for each cell line were performed in triplicate. Three 96-well plates plus an additional 4 wells of a separate 96-well control plate for determining baseline luminescence were seeded with 2000 cells/well of a particular cell line in a total volume of 90µl of a suitable growth medium for that cell line, e.g., RPMI 1640 medium supplemented with 10% FBS and any cell line specific reagents need for growth. The plates were incubated overnight at 37°C in a 5% CO₂ atmosphere.

To each of the designated baseline wells, 30µl of Cell-Titer Glo reagent (CTG; Promega Corporation) was added to each well and the plates were incubated for 20 min with shaking at room temperature. Baseline luminescence was quantitated using a BMG ClarioStar multimode plate reader according to the manufacturer's instructions.

A series of working stock 1000X drug dilutions in 100% DMSO was prepared that includes an 8 point single agent dilution of the KRas G12C inhibitor of the invention and a 5-point single agent dilution of the CDK 4/6 inhibitor of the invention. The dilutions used for the KRas G12C inhibitor and the CDK 4/6 inhibitor varied for each individual compound but were in the range of 3- to 6-fold/serial dilution.

KRas G12C inhibitors tested in this Example included:

| **Example No.*** | **Structure** |
|---|---|
| 234 | 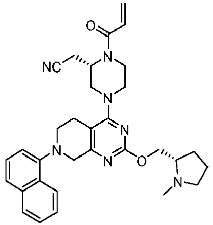 |
| 359 | 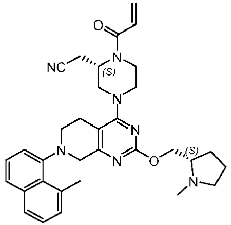 |
| 478 | 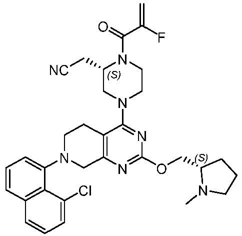 |
| 507 | 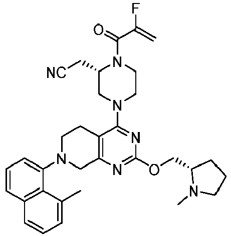 |

| | |
|---|---|
| *Example Number refers to the example number for each compound as disclosed in pending published PCT application WO2019099524. | |

A 10X intermediate dosing plate was prepared in serum free RPMI medium that contains arrayed single agent dilutions of KRas G12C inhibitor of the invention or the CDK 4/6 inhibitor of the invention. In addition, a matrix of 40 dilution combinations of KRas G12C inhibitor of the invention and the CDK 4/6 inhibitor of the invention was prepared as test samples.

To each corresponding well of the three 96-well plates seeded with the appropriate cell line above, 10µl of each 10X single agent and the 40 combinations of the dose matrix was added and the plates were incubated for 72 hours at 37C in 5% CO₂ atmosphere. A 30µl aliquot of Cell-Titer Glo reagent (CTG) was added to each test well, the plates were incubated for 20 min with shaking at room temperature, and luminescence was quantitated using a BMG ClarioStar multimode plate reader according to the manufacturer's instructions.

The raw data and metadata files were used as input files to calculate percent effect for each treatment condition and analyzed using four independent mathematical reference models designed to determine whether the two test compounds demonstrate synergy: Loewe additivity, Bliss independence, Highest Single Agent and ZIP.

The output of the data from each mathematical model is the assignment of a relative synergy score. The data reported in Table 1 are the aggregate sum of the Loewe additivity, Bliss independence, Highest Single Agent and ZIP scores ("Composite Synergy Score").

**Table 1**

| Composite Synergy Scores for CDK 4/6 Inhibitors Combined with KRas G12C Inhibitors of the invention Against KRas G12C Cell Lines | | | | |
|---|---|---|---|---|
| **CDK 4/6 Inhibitor** | **Palbociclib** | **Palbociclib** | **Palbociclib** | **Abemaciclib** |
| **KRas G12C Example #** | **234** | **478** | **478** | **478** |
| **Cell Line** | | | | |
| H1373 | -10.0 | 18.5 | 16.3 | 25.7 |
| H1792 | 39.8 | 30.7 | 24.7 | 24.4 |
| H2030 | 24.2 | 13.4 | 17.4 | 20.8 |
| H2122 | 2.8 | 25.5 | 23.1 | 13.8 |
| HCC1171 | -5.1 | -10.3 | 11.7 | 34.8 |
| HCC44 | 1.5 | 46.9 | 14.2 | 24.1 |
| LU99 | 31.6 | 29.8 | 30.9 | 26.0 |
| SW1573 | -14.7 | 29.9 | 40.7 | 16.4 |
| SW837 | -23.9 | 22.3 | 31.2 | 20.4 |

A composite score of greater than or equal to 27 was interpreted as a synergistic hit whereas a composite score between 17 and 26 indicates potential synergy. These results demonstrate that a synergistic effect was observed for the combination of a variety of CDK 4/6 family inhibitors with KRas G12C inhibitor compounds of the invention in a majority of cell lines in Table 1 that are less sensitive to KRas G12C single agent treatment harboring a KRas G12C mutation thereby increasing the sensitivity of the KRas G12C cell line to the KRas G12C inhibitor combination.

### EXAMPLE B

### In Vivo Models for Examining KRas G12C inhibitor Plus CDK 4/6 Inhibitor Combinations

Immunocompromised nude/nude mice were inoculated in the right hind flank with cells or patient derived tumor samples harboring a KRas G12C mutation. When tumor volumes reached between 200 - 400 mm³ in size, the mice were divided into four groups of 5-12 mice each. The first group was administered vehicle only. The second group was administered a single agent dose of the KRas G12C inhibitor at a concentration that yields a maximal biological effect or a less than maximal biological effect, depending on the cell line and the single agent activity, that does not result in complete tumor regression. The third group was administered a single agent dose of the CDK 4/6 inhibitor at a concentration that yields a maximal biological effect or a less than maximal biological effect, depending on the cell line and the single agent activity, that also does not result in complete tumor regression. The fourth group was administered the single agent dose of the KRas G12C inhibitor in combination with the single agent dose of the CDK 4/6 inhibitor. The treatment period varies from cell line to cell line but typically is between 21-35 days. Tumor volumes were measured using a caliper every two - three days and tumor volumes are calculated by the formula: 0.5 x (Length x Width)². A greater degree of tumor growth inhibition for the combination in this model demonstrated that the combination therapy is likely to have a clinically meaningful benefit to treated subjects relative to treatment with only a KRas G12C inhibitor.

For example, 28 nude/nude mice were inoculated in the right hind limb with 5 × 10⁶ SW1573 cells. When tumor volume reached -350 mm³ (Study Day 0), 7 mice in each of the four groups were administered p.o. daily for 28 days: vehicle only (10% Captisol in 50mM citrate buffer pH 5.0), 100 mg/kg of KRas G12C inhibitor Compound 478 (10% Captisol in 50 mM citrate buffer, pH 5.0), 130 mg/kg of the CDK 4/6 inhibitor palbociclib (saline), or 100 mg/kg of KRas G12C inhibitor Compound 478 and 130 mg/kg of palbociclib. Tumor volumes, measured at pre-specified days, for the seven mice per group were averaged and are reported in Table 2.

**Table 2**

| Average Tumor Volumes (mm³) of SW1573 Tumor Bearing Mice Treated with Single Agents and in Combination | | | | |
|---|---|---|---|---|
| **Study Day** | **Vehicle** | **Compound 478 (100mg/kg)** | **Palbociclib (130 mg/kg)** | **Compound 478 + Palbociclib Combination** |
| 0 | 353 | 357 | 356 | 356 |
| 3 | 349 | 443 | 444 | 408 |
| 6 | 403 | 404 | 484 | 298 |
| 9 | 407 | 435 | 508 | 295 |
| 11 | 489 | 444 | 515 | 300 |
| 14 | 643 | 523 | 748 | 257 |
| 16 | 691 | 575 | 807 | 248 |
| 18 | 927 | 607 | 706 | 194 |
| 21 | 1142 | 754 | 926 | 188 |
| 23 | 1239 | 758 | 1029 | 172 |
| 25 | 1468 | 741 | 1167 | 178 |
| 28 | 1635 | 785 | 1240 | 150 |

As shown in Table 2, the administration of Compound 478 or palbociclib as a single agent exhibited 71% and 40% tumor growth inhibition at Day 28, respectively. The combination of the CDK 4/6 inhibitor palbociclib and Compound 478 resulted in 58% tumor regression at Day 28.

Similarly, 20 nude/nude mice were inoculated in the right hind limb with 5 × 10⁶ H2122 cells. When tumor volume reached -325 mm³ (Study Day 0), 5 mice in each of the four groups were administered p.o. daily for 21 days: vehicle only (10% Captisol in 50mM citrate buffer pH5.0), 100 mg/kg of KRas G12C inhibitor Compound 478 (10% Captisol in 50 mM citrate buffer, pH 5.0), 130 mg/kg of the CDK 4/6 inhibitor palbociclib (saline), or 100 mg/kg of KRas G12C inhibitor Compound 478 and 130 mg/kg of palbociclib. Tumor volumes, measured at pre-specified days, for the five mice per group were averaged and are reported in Table 3.

**Table 3**

| Average Tumor Volumes (mm³) of H2122 Tumor Bearing Mice Treated with Single Agents and in Combination | | | | |
|---|---|---|---|---|
| **Study Day** | **Vehicle** | **Compound 478 (100mg/kg)** | **Palbociclib (130 mg/kg)** | **Compound 478 + Palbociclib Combination** |
| 0 | 325 | 325 | 326 | 331 |
| 2 | 520 | 340 | 385 | 338 |
| 4 | 689 | 338 | 493 | 296 |
| 7 | 955 | 381 | 601 | 254 |
| 9 | 1127 | 462 | 646 | 203 |
| 11 | 1351 | 480 | 663 | 207 |
| 14 | 1505 | 493 | 748 | 178 |
| 16 | 1574 | 541 | 765 | 169 |
| 18 | 1619 | 537 | 790 | 151 |
| 21 | 1708 | 547 | 836 | 141 |
| 23 | 1766 | 547 | 834 | 144 |

As shown in Table 3, the administration of Compound 478 or palbociclib as a single agent exhibited 85% and 65% tumor growth inhibition at Day 23, respectively. The combination of the CDK 4/6 inhibitor palbociclib and Compound 478 resulted in 56% tumor regression at Day 23.

In another experiment, on Day 0, 20 nude/nude mice were inoculated in the right hind limb with 5 × 10⁶ LU6405 cells. When tumor volume reached ~350 mm³ (Study Day 1), 5 mice in each of the four groups were administered p.o. daily for 21 days: vehicle only (10% Captisol in 50mM citrate buffer pH5.0), 100 mg/kg of KRas G12C inhibitor Compound 478 (10% Captisol in 50 mM citrate buffer, pH 5.0), 130 mg/kg of the CDK 4/6 inhibitor palbociclib (saline), or 100 mg/kg of KRas G12C inhibitor Compound 478 and 130 mg/kg of palbociclib. Tumor volumes, measured at pre-specified days, for the five mice per group were averaged and are reported in Table 4.

**Table 4**

| Average Tumor Volumes (mm³) of LU6405 Tumor Bearing Mice Treated with Single Agents and in Combination | | | | |
|---|---|---|---|---|
| **Study Day** | **Vehicle** | **Compound 478 (100mg/kg)** | **Palbociclib (130 mg/kg)** | **Compound 478 - Palbociclib Combination** |
| 1 | 256.45 | 256.06 | 255.36 | 257.48 |
| 4 | 447.44 | 308.98 | 440.79 | 275.54 |
| 8 | 877.95 | 324.46 | 659.01 | 267.08 |
| 11 | 1215.89 | 329.09 | 834.95 | 238.21 |
| 15 | 1727.56 | 310.21 | 1165.80 | 154.62 |
| 18 | 1950.09 | 290.88 | 1295.57 | 111.00 |
| 22 | 2074.33 | 257.34 | 1363.40 | 59.38 |
| 24 | 2074.33 | 249.19 | 1382.59 | 54.85 |

As shown in Table 4, the administration of Compound 478 exhibited 96% tumor growth inhibition at Day 24. The combination of the CDK 4/6 inhibitor palbociclib and Compound 478 resulted in 77% tumor regression at Day 24.

These results demonstrate that the combination therapy resulted in greater amount of tumor growth inhibition compared to either single agent alone demonstrating enhanced in vivo anti-tumor efficacy of the combination against KRas G12C expressing cancer.

## Claims

1. A combination of a CDK 4/6 inhibitor and a KRAS G12C inhibitor for use in a method of treating cancer in a subject in need thereof, comprising administering to the subject a therapeutically effective amount of the combination of a CDK 4/6 inhibitor and a KRAS G12C inhibitor,
wherein the CDK 4/6 inhibitor is abemaciclib or palbociclib, and
wherein the KRas G12C inhibitor is selected from the group consisting of: 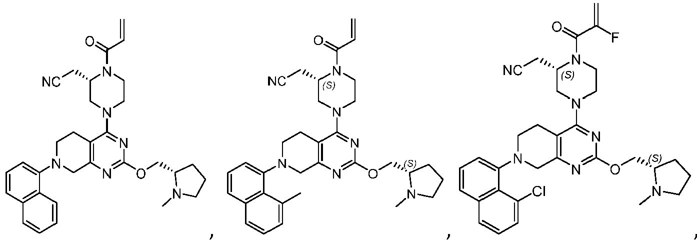 and 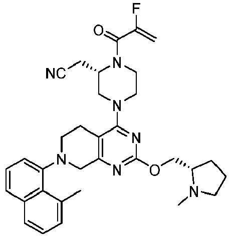 or pharmaceutically acceptable salts thereof.

2. The combination of a CDK 4/6 inhibitor and a KRAS G12C inhibitor for use according to claim 1, wherein the KRas G12C inhibitor is: 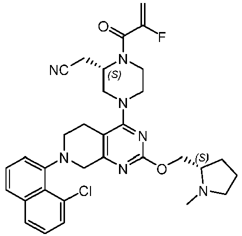 or a pharmaceutically acceptable salt thereof.

3. The combination of a CDK 4/6 inhibitor and a KRAS G12C inhibitor for use according to claim 1 or 2, wherein the therapeutically effective amount of the combination of the CDK 4/6 inhibitor and the KRAS G12C inhibitor results in an increased duration of overall survival, an increased duration of progression free survival, an increase in tumor growth regression, an increase in tumor growth inhibition or an increased duration of stable disease in the subjects relative to treatment with only the KRas G12C inhibitor.

4. A pharmaceutical composition, comprising a therapeutically effective amount of a combination of a CDK 4/6 inhibitor and a KRas G12C inhibitor according to claim 1 or 2, and a pharmaceutically acceptable excipient.

5. An in vitro method for inhibiting KRas G12C activity in a cell, comprising contacting the cell in which inhibition of KRas G12C activity is desired with an effective amount of a CDK 4/6 inhibitor and a KRas G12C inhibitor compound according to claim 1 or 2, pharmaceutical compositions or pharmaceutically acceptable salts thereof, wherein the CDK 4/6 inhibitor synergistically increases the sensitivity of the cancer cells to the KRas G12C inhibitor.

6. A CDK 4/6 inhibitor and a KRas G12C inhibitor compound according to claim 1 or 2, pharmaceutical compositions or pharmaceutically acceptable salts thereof for use in a method for inhibiting KRas G12C activity in a cell, comprising contacting the cell in which inhibition of KRas G12C activity is desired with an effective amount of the CDK 4/6 inhibitor and the KRas G12C inhibitor compound according to claim 1 or 2, pharmaceutical compositions or pharmaceutically acceptable salts thereof, wherein the CDK 4/6 inhibitor synergistically increases the sensitivity of the cancer cells to the KRas G12C inhibitor.

7. The combination of a CDK 4/6 inhibitor and a KRAS G12C inhibitor for use according to claim 1 or 2, wherein the CDK 4/6 inhibitor synergistically increases the sensitivity of the cancer cells to the KRas G12C inhibitor.

8. A CDK 4/6 inhibitor for use in a method for increasing the sensitivity of a cancer cell to a KRas G12C inhibitor compound comprising administering to a subject undergoing KRas G12C treatment with a compound according to claim 1 or 2 or a pharmaceutically acceptable salt thereof, alone or combined with a pharmaceutically acceptable carrier, excipient or diluents, a therapeutically effective amount of a CDK 4/6 inhibitor according to claim 1 or 2, wherein the CDK 4/6 inhibitor synergistically increases the sensitivity of the cancer cell to the KRas G12C inhibitor.

9. The combination of a CDK 4/6 inhibitor and a KRAS G12C inhibitor for use according to any one of claims 1, 2, 6 and 7, the method according to claim 5, or the CDK 4/6 inhibitor for use according to claim 8, wherein the therapeutically effective amount of the KRas G12C inhibitor in the combination is between about 0.01 to 100 mg/kg per day.

10. The combination of a CDK 4/6 inhibitor and a KRAS G12C inhibitor for use according to any one of claims 1-3, 6, 7 and 9, the method according to any one of claims 5 and 9, or the CDK 4/6 inhibitor for use according to claim 8 or 9, wherein the cancer is a KRas G12C-associated cancer selected from the group consisting of Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hamartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Biliary tract: gall bladder carcinoma, ampullary carcinoma, cholangiocarcinoma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiform, oligodendroglioma, schwannoma, retinoblastoma, congenital tumors), spinal cord neurofibroma, meningioma, glioma, sarcoma); Gynecological: uterus (endometrial carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), fallopian tubes (carcinoma); Hematologic: blood (myeloid leukemia (acute and chronic), acute lymphoblastic leukemia, chronic lymphocytic leukemia, myeloproliferative diseases, multiple myeloma, myelodysplastic syndrome), Hodgkin's disease, non-Hodgkin's lymphoma (malignant lymphoma); Skin: malignant melanoma, basal cell carcinoma, squamous cell carcinoma, Kaposi's sarcoma, moles dysplastic nevi, lipoma, angioma, dermatofibroma, keloids, psoriasis; and Adrenal glands: neuroblastoma.

11. The combination of a CDK 4/6 inhibitor and a KRAS G12C inhibitor for use according to claim 10, the method according to claim 10, or the CDK 4/6 inhibitor for use according to claim 10, wherein the cancer is non-small cell lung cancer.

12. A kit comprising the pharmaceutical composition of claim 4 for treating KRas G12C cancer in a subject.

13. A kit comprising: a) a pharmaceutical composition comprising a CDK 4/6 inhibitor and b) a pharmaceutical composition comprising a KRas G12C inhibitor for treating a KRas G12C cancer in a subject,
wherein the CDK 4/6 inhibitor is abemaciclib or palbociclib, and
wherein the KRas G12C inhibitor is selected from the group consisting of: 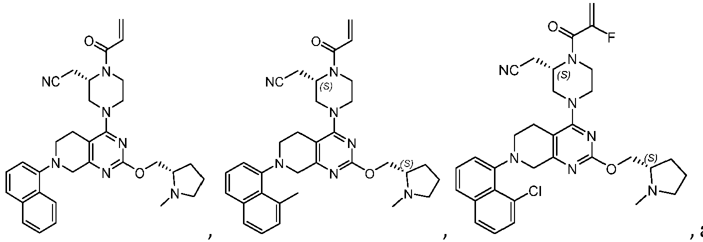 and 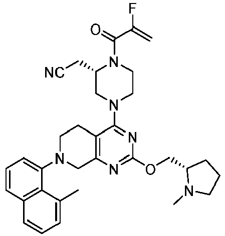 or pharmaceutically acceptable salts thereof.

## Patentansprüche

1. Kombination aus einem CDK-4/6-Inhibitor und einem KRAS-G12C-Inhibitor zur Verwendung in einem Verfahren zum Behandeln von Krebs bei einem Subjekt, das dessen bedarf, umfassend Verabreichen einer therapeutisch wirksamen Menge der Kombination aus einem CDK-4/6-Inhibitor und einem KRAS-G12C-Inhibitor an das Subjekt,
wobei der CDK-4/6-Inhibitor Abemaciclib oder Palbociclib ist, und
wobei der KRas-G12C-Inhibitor ausgewählt ist aus der Gruppe bestehend aus: 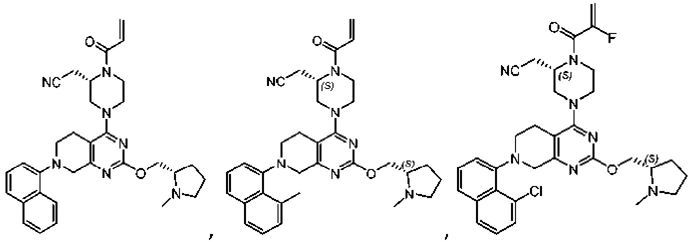 und 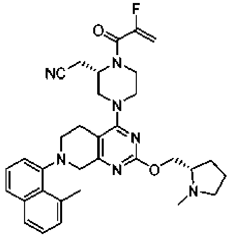 oder pharmazeutisch verträgliche Salze davon.

2. Kombination aus einem CDK-4/6-Inhibitor und einem KRAS-G12C-Inhibitor zur Verwendung gemäß Anspruch 1, wobei der KRas-G12C-Inhibitor wie folgt ist: 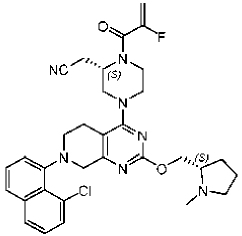 oder pharmazeutisch verträgliches Salz davon.

3. Kombination aus einem CDK-4/6-Inhibitor und einem KRAS-G12C-Inhibitor zur Verwendung gemäß Anspruch 1 oder 2, wobei die therapeutisch wirksame Menge der Kombination aus dem CDK-4/6-Inhibitor und dem KRAS-G12C-Inhibitor zu einer erhöhten Dauer an Gesamtüberleben, einer erhöhten Dauer an progressionsfreiem Überleben, einer Erhöhung der Tumorwachstumsregression, einer Erhöhung der Tumorwachstumshemmung oder einer erhöhten Dauer an stabiler Erkrankung bei den Subjekten relativ zu Behandlung mit nur dem KRas-G12C-Inhibitor führt.

4. Pharmazeutische Zusammensetzung, umfassend eine therapeutisch wirksame Menge einer Kombination aus einem CDK-4/6-Inhibitor und einem KRas-G12C-Inhibitor gemäß Anspruch 1 oder 2 und einen pharmazeutisch verträglichen Träger.

5. In-vitro-Verfahren zum Hemmen von KRas-G12C-Aktivität in einer Zelle, umfassend Kontaktieren der Zelle, in der Hemmung von KRas-G12C-Aktivität erwünscht ist, mit einer wirksamen Menge aus einem CDK-4/6-Inhibitor und einer KRas-G12C-Inhibitorverbindung gemäß Anspruch 1 oder 2, pharmazeutischen Zusammensetzungen oder pharmazeutisch verträglichen Salzen davon, wobei der CDK-4/6-Inhibitor die Empfindlichkeit der Krebszellen gegenüber dem KRas-G12C-Inhibitor synergistisch erhöht.

6. CDK-4/6-Inhibitor und KRas-G12C-Inhibitorverbindung gemäß Anspruch 1 oder 2, pharmazeutische Zusammensetzungen oder pharmazeutisch verträgliche Salze davon zur Verwendung in einem Verfahren zum Hemmen von KRas-G12C-Aktivität in einer Zelle, umfassend Kontaktieren der Zelle, in der Hemmung von KRas-G12C-Aktivität erwünscht ist, mit einer wirksamen Menge des CDK-4/6-Inhibitors und der KRas-G12C-Inhibitorverbindung gemäß Anspruch 1 oder 2, pharmazeutischen Zusammensetzungen oder pharmazeutisch verträglichen Salzen davon, wobei der CDK-4/6-Inhibitor die Empfindlichkeit der Krebszellen gegenüber dem KRas-G12C-Inhibitor synergistisch erhöht.

7. Kombination aus einem CDK-4/6-Inhibitor und einem KRAS-G12C-Inhibitor zur Verwendung gemäß Anspruch 1 oder 2, wobei der CDK-4/6-Inhibitor die Empfindlichkeit der Krebszellen gegenüber dem KRas-G12C-Inhibitor synergistisch erhöht.

8. CDK-4/6-Inhibitor zur Verwendung in einem Verfahren zum Erhöhen der Empfindlichkeit einer Krebszelle gegenüber einer KRas-G12C-Inhibitorverbindung, umfassend Verabreichen, an ein Subjekt, das KRas-G12C-Behandlung mit einer Verbindung gemäß Anspruch 1 oder 2 oder einem pharmazeutisch verträglichen Salz davon erfährt, alleine oder kombiniert mit einem pharmazeutisch verträglichen Träger, Hilfsstoff oder Verdünnungsmitteln, einer therapeutisch wirksamen Menge eines CDK-4/6-Inhibitors gemäß Anspruch 1 oder 2, wobei der CDK-4/6-Inhibitor die Empfindlichkeit der Krebszelle gegenüber dem KRas-G12C-Inhibitor synergistisch erhöht.

9. Kombination aus einem CDK-4/6-Inhibitor und einem KRAS-G12C-Inhibitor zur Verwendung gemäß einem der Ansprüche 1, 2, 6 und 7, Verfahren gemäß Anspruch 5 oder CDK-4/6-Inhibitor zur Verwendung gemäß Anspruch 8, wobei die therapeutisch wirksame Menge des KRas-G12C-Inhibitors in der Kombination zwischen etwa 0,01 bis 100 mg/kg pro Tag ist.

10. Kombination aus einem CDK-4/6-Inhibitor und einem KRAS-G12C-Inhibitor zur Verwendung gemäß einem der Ansprüche 1-3, 6, 7 und 9, Verfahren gemäß einem der Ansprüche 5 und 9 oder CDK-4/6-Inhibitor zur Verwendung gemäß Anspruch 8 oder 9, wobei der Krebs ein KRas-G12C-assoziierter Krebs ist, ausgewählt aus der Gruppe bestehend aus Herz: Sarkom (Angiosarkom, Fibrosarkom, Rhabdomyosarkom, Liposarkom), Myxom, Rhabdomyom, Fibrom, Lipom und Teratom; Lunge: bronchogenes Karzinom (squamöse Zelle, undifferenzierte kleine Zelle, undifferenzierte große Zelle, Adenokarzinom), alveolares (bronchiolares) Karzinom, bronchiales Adenom, Sarkom, Lymphom, chondromatöses Hamartom, Mesotheliom; gastrointestinal: Ösophagus (squamöses Zellkarzinom, Adenokarzinom, Leiomyosarkom, Lymphom), Bauch (Karzinom, Lymphom, Leiomyosarkom), Bauchspeicheldrüse (duktales Adenokarzinom, Insulinom, Glucagonom, Gastrinom, Karzinoidtumoren, Vipom), Dünndarm (Adenokarzinom, Lymphom, Karzinoidtumoren, Kaposi-Sarkom, Leiomyom, Hemangiom, Lipom, Neurofibrom, Fibrom), Dickdarm (Adenokarzinom, tubulares Adenom, bösartiges Adenom, Hamartom, Leiomyom); Urogenitaltrakt: Niere (Adenokarzinom, Wilm-Tumor (Nephroblastom), Lymphom, Leukämie), Blase und Urethra (squamöses Zellkarzinom, Übergangszellkarzinom, Adenokarzinom), Prostata (Adenokarzinom, Sarkom), Testis (Seminom, Teratom, embryonales Karzinom, Teratokarzinom, Choriokarzinom, Sarkom, interstitielles Zellkarzinom, Fibrom, Fibroadenom, adenomatoide Tumoren, Lipom); Leber: Hepatom (hepatozelluläres Karzinom), Cholangiokarzinom, Hepatoblastom, Angiosarkom, hepatozelluläres Adenom, Hemangiom; Gallengang: Gallenblasenkarzinom, ampulläres Karzinom, Cholangiokarzinom; Knochen: osteogenes Sarkom (Osteosarkom), Fibrosarkom, malignes fibröses Histiozytom, Chondrosarkom, Ewing-Sarkom, malignes Lymphom (Retikulumzellensarkom), multiples Myelom, malignes Riesenzellentumorchordom, Osteochronfrom (osteokartilaginäre Exostosen), benignes Chondrom, Chondroblastom, Chondromyxofibrom, osteoides Osteom und Riesenzellentumore; Nervensystem: Schädel (Osteom, Hemangiom, Granulom, Xanthom, Osteitis deformans), Meningen (Meningiom, Meningiosarkom, Gliomatose), Hirn (Astrozytom, Medulloblastom, Gliom, Ependymom, Germinom (Pinealom), Glioblastom multiform, Oligodendrogliom, Schwannom, Retinoblastom, kongenitale Tumoren), Rückenmarksneurofibrom, Meningiom, Gliom, Sarkom); gynäkologisch: Uterus (endometriales Karzinom (seröses Cystadenokarzinom, muzinöses Cystadenokarzinom, unklassifiziertes Karzinom), Granulosa-thekale Zelltumoren, Sertoli-Leydig-Zelltumoren, Dysgerminom, malignes Teratom), Vulva (squamöses Zellkarzinom, intraepitheliales Karzinom, Adenokarzinom, Fibrosarkom, Melanom), Vagina (klares Zellkarzinom, squamöses Zellkarzinom, botryoides Sarkom (embryonales Rhabdomyosarkom), Eileiter (Karzinom); hämatologisch: Blut (myeloische Leukämie (akut und chronisch), akute lymphoblastische Leukämie, chronische lymphozytische Leukämie, myeloproliferative Erkrankungen, multiples Myelom, myelodysplastisches Syndrom), Morbus Hodgkin, Nicht-Hodgkin-Lymphom (malignes Lymphom); Haut: malignes Melanom, Basalzellenkarzinom, squamöses Zellkarzinom, Kaposi-Sarkom, Molen, dysplastische Muttermale, Lipom, Angiom, Dermatofibrom, Keloide, Psoriasis; und Nebennieren: Neuroblastom.

11. Kombination aus einem CDK-4/6-Inhibitor und einem KRAS-G12C-Inhibitor zur Verwendung gemäß Anspruch 10, Verfahren gemäß Anspruch 10 oder CDK-4/6-Inhibitor zur Verwendung gemäß Anspruch 10, wobei der Krebs nicht-kleinzelliger Lungenkrebs ist.

12. Kit, umfassend die pharmazeutische Zusammensetzung nach Anspruch 4 zum Behandeln von KRas-G12C-Krebs bei einem Subjekt.

13. Kit, umfassend: a) eine pharmazeutische Zusammensetzung, umfassend einen CDK-4/6-Inhibitor und b) eine pharmazeutische Zusammensetzung, umfassend einen KRas-G12C-Inhibitor zum Behandeln eines KRas-G12C-Krebses bei einem Subjekt,
wobei der CDK-4/6-Inhibitor Abemaciclib oder Palbociclib ist, und
wobei der KRas-G12C-Inhibitor ausgewählt ist aus der Gruppe bestehend aus: 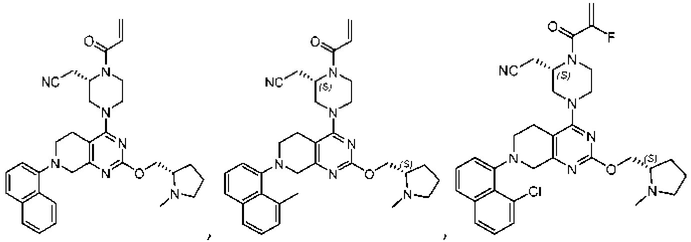 und 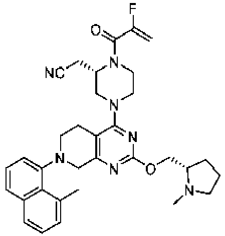 oder pharmazeutisch verträgliche Salze davon.

## Revendications

1. Combinaison d'un inhibiteur de CDK 4/6 et d'un inhibiteur de KRAS G12C destinée à être utilisée dans une méthode de traitement d'un cancer chez un sujet en ayant besoin, comprenant l'administration au sujet d'une quantité thérapeutiquement efficace de la combinaison d'un inhibiteur de CDK 4/6 et d'un inhibiteur de KRAS G12C,
ledit inhibiteur de CDK 4/6 étant l'abémaciclib ou le palbociclib, et
ledit inhibiteur de KRas G12C étant choisi dans le groupe constitué par : 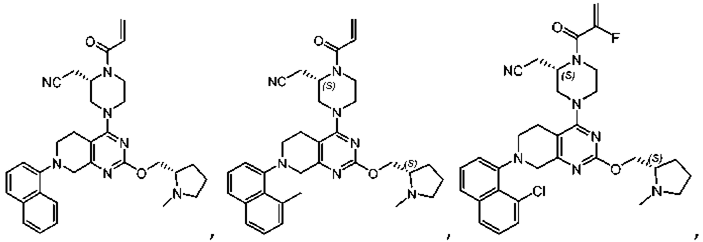 et 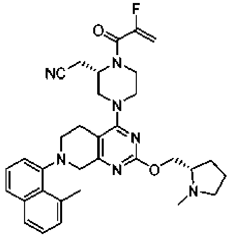 ou les sels pharmaceutiquement acceptables de ceux-ci.

2. Combinaison d'un inhibiteur de CDK 4/6 et d'un inhibiteur de KRAS G12C destinée à être utilisée selon la revendication 1, ledit inhibiteur de KRas G12C étant : 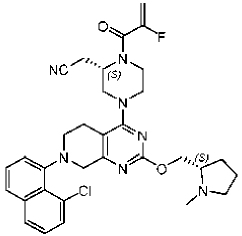 ou un sel pharmaceutiquement acceptable de celui-ci.

3. Combinaison d'un inhibiteur de CDK 4/6 et d'un inhibiteur de KRAS G12C destinée à être utilisée selon la revendication 1 ou 2, ladite quantité thérapeutiquement efficace de la combinaison de l'inhibiteur de CDK 4/6 et de l'inhibiteur de KRAS G12C conduisant à une durée accrue de la survie globale, une durée accrue de la survie sans progression, une augmentation de la régression de la croissance tumorale, une augmentation de l'inhibition de la croissance tumorale ou une durée accrue de maladie stable chez les sujets par rapport à un traitement avec uniquement l'inhibiteur de KRas G12C.

4. Composition pharmaceutique comprenant une quantité thérapeutiquement efficace d'une combinaison d'un inhibiteur de CDK 4/6 et d'un inhibiteur de KRas G12C selon la revendication 1 ou 2, et un excipient pharmaceutiquement acceptable.

5. Méthode in vitro permettant l'inhibition de l'activité KRas G12C dans une cellule, comprenant la mise en contact de la cellule dans laquelle l'inhibition de l'activité KRas G12C est souhaitée avec une quantité efficace d'un inhibiteur de CDK 4/6 et d'un composé inhibiteur de KRas G12C selon la revendication 1 ou 2, de compositions pharmaceutiques ou de sels pharmaceutiquement acceptables de ceux-ci, ledit inhibiteur de CDK 4/6 augmentant de manière synergique la sensibilité des cellules cancéreuses à l'inhibiteur de KRas G12C.

6. Inhibiteur de CDK 4/6 et composé inhibiteur de KRas G12C selon la revendication 1 ou 2, compositions pharmaceutiques ou sels pharmaceutiquement acceptables de ceux-ci, destinés à être utilisés dans une méthode permettant d'inhiber l'activité KRas G12C dans une cellule, comprenant la mise en contact de la cellule dans laquelle l'inhibition de l'activité KRas G12C est souhaitée avec une quantité efficace de l'inhibiteur de CDK 4/6 et du composé inhibiteur de KRas G12C selon la revendication 1 ou 2, de compositions pharmaceutiques ou de sels pharmaceutiquement acceptables de ceux-ci, ledit inhibiteur de CDK 4/6 augmentant de manière synergique la sensibilité des cellules cancéreuses à l'inhibiteur de KRas G12C.

7. Combinaison d'un inhibiteur de CDK 4/6 et d'un inhibiteur de KRAS G12C destinée à être utilisée selon la revendication 1 ou 2, ledit inhibiteur de CDK 4/6 augmentant de manière synergique la sensibilité des cellules cancéreuses à l'inhibiteur de KRas G12C.

8. Inhibiteur de CDK 4/6 destiné à être utilisé dans une méthode permettant d'augmenter la sensibilité d'une cellule cancéreuse à un composé inhibiteur de KRas G12C comprenant l'administration à un sujet suivant un traitement de KRas G12C avec un composé selon la revendication 1 ou 2 ou un sel pharmaceutiquement acceptable de celui-ci, seul ou combiné à un vecteur, un excipient ou des diluants pharmaceutiquement acceptables, d'une quantité thérapeutiquement efficace d'un inhibiteur de CDK 4/6 selon la revendication 1 ou 2, ledit inhibiteur de CDK 4/6 augmentant de manière synergique la sensibilité de la cellule cancéreuse à l'inhibiteur de KRas G12C.

9. Combinaison d'un inhibiteur de CDK 4/6 et d'un inhibiteur de KRAS G12C destinée à être utilisée selon l'une quelconque des revendications 1, 2, 6 et 7, méthode selon la revendication 5, ou inhibiteur de CDK 4/6 destiné à être utilisé selon la revendication 8, ladite quantité thérapeutiquement efficace de l'inhibiteur de KRas G12C dans la combinaison étant comprise entre environ 0,01 à 100 mg/kg par jour.

10. Combinaison d'un inhibiteur de CDK 4/6 et d'un inhibiteur de KRAS G12C destinée à être utilisée selon l'une quelconque des revendications 1 à 3, 6, 7 et 9, méthode selon l'une quelconque des revendications 5 et 9, ou inhibiteur de CDK 4/6 destiné à être utilisé selon la revendication 8 ou 9, ledit cancer étant un cancer associé à KRas G12C choisi dans le groupe constitué par Cardiaque : sarcome (angiosarcome, fibrosarcome, rhabdomyosarcome, liposarcome), myxome, rhabdomyome, fibrome, lipome et tératome ; Poumon : carcinome bronchogène (cellule squameuse, petite cellule indifférenciée, grande cellule indifférenciée, adénocarcinome), carcinome alvéolaire (bronchiolaire), adénome, sarcome, lymphome, hamartome chondromateux, mésothéliome bronchiques ; Gastrointestinal : oesophage (carcinome, adénocarcinome, léïomyosarcome, lymphome à cellules squameuses), estomac (carcinome, lymphome, léïomyosarcome), pancréas (adénocarcinome, insulinome, glucagonome, gastrinome, tumeurs carcinoïdes, vipome ductaux), intestin grêle (adénocarcinome, lymphome, tumeurs carcinoïdes, sarcome de Kaposi, léiomyome, hémangiome, lipome, neurofibrome, fibrome), gros intestin (adénocarcinome, adénome tubulaire, adénome villeux, hamartome, léïomyome) ; Tractus génito-urinaire : rein (adénocarcinome, tumeur de Wilm (néphroblastome), lymphome, leucémie), vessie et urètre (carcinome à cellules squameuses, carcinome à cellules transitionnelles, adénocarcinome), prostate (adénocarcinome, sarcome), testicules (séminome, tératome, carcinome embryonnaire, tératocarcinome, choriocarcinome, sarcome, carcinome à cellules interstitielles, fibrome, fibroadénome, tumeurs adénomatoïdes, lipome) ; Foie : hépatome (carcinome hépatocellulaire), cholangiocarcinome, hépatoblastome, angiosarcome, adénome hépatocellulaire, hémangiome ; Tractus biliaire : carcinome de la vésicule biliaire, carcinome ampullaire, cholangiocarcinome ; Os : sarcome ostéogène (ostéosarcome), fibrosarcome, histiocytome fibreux malin, chondrosarcome, sarcome d'Ewing, lymphome malin (sarcome à cellules réticulaires), myélome multiple, tumeur maline à cellules géantes chordome, ostéochronfrome (exostoses ostéocartilagineuses), chondrome bénin, chondroblastome, chondromyxofibrome, ostéome ostéoïde et tumeurs à cellules géantes ; Système nerveux : crâne (ostéome, hémangiome, granulome, xanthome, ostéite déformante), méninges (méningiome, méningiosarcome, gliomatose), cerveau (astrocytome, médulloblastome, gliome, épendymome, germinome (pinéalome), glioblastome multiforme, oligodendrogliome, schwannome, rétinoblastome, tumeurs congénitales), moelle épinière (neurofibrome, méningiome, gliome, sarcome) ; Gynécologique : utérus (carcinome de l'endomètre (cystadénocarcinome séreux, cystadénocarcinome mucineux, carcinome non classifié), tumeurs à cellules de la granulosa et thécales, tumeurs à cellules de Sertoli-Leydig, dysgerminome, tératome malin), vulve (carcinome à cellules squameuses, carcinome intraépithélial, adénocarcinome, fibrosarcome, mélanome), vagin (carcinome à cellules claires, carcinome à cellules squameuses, sarcome botryoïde (rhabdomyosarcome embryonnaire), trompes de Fallope (carcinome) ; Hématologique : sang (leucémie myéloïde (aiguë et chronique), leucémie lymphoblastique aiguë, leucémie lymphocytaire chronique, maladies myéloprolifératives, myélome multiple, syndrome myélodysplasique), maladie de Hodgkin, lymphome non-hodgkinien (lymphome malin) ; Peau : mélanome malin, carcinome à cellules basales, carcinome à cellules squameuses, sarcome de Kaposi, naevus dysplasiques de grains de beauté, lipome, angiome, dermatofibrome, chéloïdes, psoriasis ; et Glandes surrénales : neuroblastome.

11. Combinaison d'un inhibiteur de CDK 4/6 et d'un inhibiteur de KRAS G12C destinée à être utilisée selon la revendication 10, méthode selon la revendication 10, ou inhibiteur de CDK 4/6 destiné à être utilisé selon la revendication 10, ledit cancer étant un cancer du poumon non à petites cellules.

12. Kit comprenant la composition pharmaceutique selon la revendication 4 permettant de traiter un cancer à KRas G12C chez un sujet.

13. Kit comprenant : a) une composition pharmaceutique comprenant un inhibiteur de CDK 4/6 et b) une composition pharmaceutique comprenant un inhibiteur de KRas G12C destinée à traiter un cancer à KRas G12C chez un sujet,
ledit inhibiteur de CDK 4/6 étant l'abémaciclib ou le palbociclib, et
ledit inhibiteur de KRas G12C étant choisi dans le groupe constitué par : 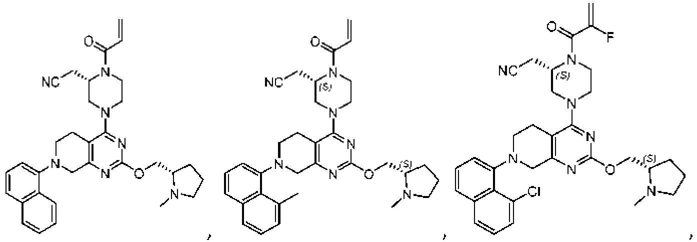 et 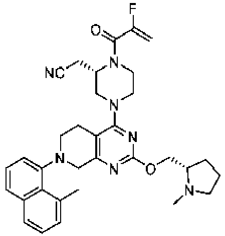 ou des sels pharmaceutiquement acceptables de ceux-ci.
